# EUROPEAN PATENT APPLICATION

(11) **EP 1 997 885 A1**
(43) Date of publication of application: **03.12.2008**
(21) Application number: 07010568.9
(22) Date of filing: 29.05.2007
(51) Int. Cl.: C12N 15/00, C07K 1/00, C07K 14/435

(54) **Fluorescent proteins and methods of use thereof**

(71) Applicant: AXXAM S.p.A., 20132 Milano (IT)
(72) Inventor: Tasdemir, Aynur, 20132 Milano (IT); Corazza, Sabrina, 20132 Milano (IT)
(74) Representative: Banfi, Paolo

(57) **Abstract**

The invention relates to dimeric and monomeric mutants of the naturally occurring tetrameric *Anemonia sulcata* Green Fluorescent Protein 499 (AsGFP499), peptides thereof and polynucleotides encoding them, and to their use as intracellular fluorescent markers particularly in the set up of cell-based or cell-free assays.

## Description

The present invention relates to dimeric and monomeric mutants of the naturally occurring tetrameric *Anemonia sulcata* Green Fluorescent Protein 499 (AsGFP499) {Wiedenmann, 2000 #27; Nienhaus, 2006 #170}. The fluorescent proteins of the invention present advantageous properties such as high expression in eucaryotic cells, decreased oligomerization tendency, improved spectra, high stability at 37°C, high water solubility and low bulkiness, all characteristics which make their use in reporter gene technology and in cell-based assays particularly attractive. The fluorescent proteins of the invention and their encoding polynucleotides are also useful for protein labelling, in the study of protein interactions, in diagnostics and medical imaging.

### BACKGROUND OF THE INVENTION

The Green Fluorescent Protein (GFP) family contains a growing number of fluorescent proteins (FP) and chromoproteins (CP). Up to now more than 100 have been cloned or modified, and many of them have been described and characterized from marine invertebrates such as from organisms of the Cnidaria phylum {Johnson, 1962 #174; Morin, 1971 #13; Wampler, 1971 #14; Morin, 1974 #16; Labas, 2002 #30; Matz, 1999 #28; Wiedenmann, 2000 #27; Lukyanov, 2000 #111; Gurskaya, 2001 #97}.

Chromoproteins generally require prosthetic groups such as small non-peptide molecules or metal ions{Biron, 2003 #169}, unlike these proteins, the proteins of the GFP family synthesize their own "prosthetic group" without need of external agents, except molecular oxygen, for gaining their optical properties {Heim, 1994 #22; Cubitt, 1995 #20; Reid, 1997 #171}.

In 1955 Davenport et al.{Davenport, 1955 #3} reported that irradiation of the pacific jellyfish *Aequorea Victoria* with long-wave ultraviolet light, caused a green fluorescence. during purification of a blue-light emitting protein from this organism Shimomura et al. {Shimomura, 1962 #173} notated a greenish light in the solution. The blue fluorescent protein was the famous photoprotein Aequorin and the green one GFP. The photoprotein is activated by calcium-ions, which bind to the three EF-hand structures, typical of calcium binding proteins, present within the protein. In the presence of these ions, Aequorin undergoes a conformational change, leading to the oxidation of bound coelenterazine with the subsequent release of CO₂ and a flash of blue light at 470nm. *Aequorea victoria* GFP (*A*νGFP) is a part of the jellyfish's bioluminescent system and plays the role of the secondary emitter, transforming the blue light from Aequorin into green light at 508nm. In fact, GFP serves as an energy-transfer acceptor by absorption of blue light at λ max = 470 nm from Ca²⁺-activated Aequorin thereupon emitting green light at λ max = 508 nm {Morise, 1974 #17; Johnson, 1962 #174}.

Prendergast et al. {Prendergast, 1978 #18} estimated the molecular weight (M_{w}) of GFP to be around 27 kDa. 40 years after the discovery of GFP, the breakthrough came with the cloning of the wild-type gene {Prasher, 1992 #5}, encoding a 238 amino acid protein with a theoretical M_{w} of 26,9 kDa, which was later expressed in different systems {Chalfie, 1994 #19}. These results indicated that GFP could be used in any organism due to the unique ability of GFP to form a chromophore by itself. The formation of its chromophore was independent on factors such as enzymes or other proteins originally coming from *Aequorea victoria.* It was revealed that the gene's protein sequence intrinsically encodes a strong chromophore, which does not require external substrates or co-factors, except for molecular oxygen {Prasher, 1992 #5; Cody, 1993 #21; Heim, 1994 #22}.

The crystal structure of GFP was independently solved in 1996 by two groups {Ormo, 1996 #25, Yang, 1996 #26}. A new protein fold was discovered, which was called β-Can {Yang, 1996 #26}. The structure reminds to a β-barrel formed by 11 anti-parallel β-sheets, which is crossed by an α-helix. The tripeptide sequence: S65-T66-G67 is located almost in the middle of the helix, serving by an autocatalytic reaction as the backbone for the chromophoric structure. The chromophore itself is a p-hydroxybenzylidene-imidazolidone ring.

GFP has become an important tool in molecular and cellular biology as a reporter of gene expression, fusion tag to monitor protein localization within living cells, tracer of cell lineage, partner for fluorescence resonance energy transfer (FRET) and biosensor {Tsien, 1998 #23; Kendall, 1998 #24}.

Despite its remarkable usefulness, wild-type GFP exhibits some limitations, such as problems with expression level, photosensitivity, low brightness, insolubility and the tendency to dimerize at high concentrations. To overcome these reported disadvantages, efforts were done to improve the wild-type GFP by mutagenesis, in order to obtain mutants, which were more soluble, photo-resistant, brighter and with shifted spectral properties.

Despite a modest or low degree of amino acid sequence identity, all GFP-like proteins are likely to share the β-Can structure{Matz, 2002 #108}{Chudakov, 2005 #106}{Evdokimov, 2006 #103}, and sequence alignments show furthermore that amino acids which are a part of the chromophore, such as T66, G67 and others which interact with the chromophore, such as R96 and E222 (numbering according GFP) are conserved in the FPs {Baird, 2000 #35}.

One great disadvantage of the Anthozoa GFP-like proteins is their propensity to oligomerize {Verkhusha, 2004 #79}. The oligomerization does not narrow their use as reporter genes or biomarkers, but hampers their use in fusion protein applications. Target proteins in fusion with them could form bulky products and interfere with their translocation or localization in cells, especially in cases where the target protein itself tends to form oligomers. Others could be even activated by oligomerization, and fusion of them with oligomeric FPs could lead to constitutively active target proteins, which could harm the host cell{Zacharias, 2002 #59}. Therefore it is crucial and necessary to find or engineer FPs, which minimally affect a target protein.

### DISCLOSURE OF THE INVENTION

In a first aspect, the invention provides an isolated fluorescent protein or polypeptide which:
(a) emits green light upon excitation with light in the range of λ 396-490 nm;
(b) has an amino acid sequence which is at least 80%, preferably at least 90%, more preferably at least 95% identical to SEQ ID NO°:1;
(c) in sequence alignment with SEQ ID NO°:1, presents one of the following multiple substitutions (the residue positions are referred to SEQ ID NO°:1):
   i) S103K
   ii) S103K, T159K and F173E
   iii) S94T, S103K, T159K and F173E
   iv) S103K, R150E
   v) S103K, F173E
   vi) S94T, S103K, R150K, S171N and K206R
   vii) S94T, S103K, R150I, S171N and K206R

The invention also includes a fragment of a protein or polypeptide as defined above, said fragment being water-soluble, retaining the capability of emitting green light upon excitation with light in the range of λ 396-490 nm and presenting at least one of the identified mutations.

In a preferred embodiment, the fluorescent protein or polypeptide according to the invention is selected from the group consisting of SEQ ID NOs.:2-8. The amino acid sequences can be modified without negatively affecting the fluorescent protein's activity, especially by conservative substitutions of amino acidic residues within the indicated sequence-identity limits. In addition, the protein sequences can be deleted of small portions, without altering the fluorescent activity.

In a preferred embodiment, the fluorescent proteins which are subjected to mutagenesis according to the invention are isolated from organisms of the species *Anemonia sulcata.* These proteins, which show very low sequence homology to known proteins, are water-soluble and temperature-stable, can be induced in bacteria at 37°C within 6 hours and, thanks to their low bulkiness, are particular suitable for translocation assays.

In another aspect the invention provides antibodies that specifically bind to the subject fluorescent proteins. Suitable antibodies are obtained by immunizing a host animal such as mouse, rat, sheep, goat, hamster, rabbit, etc., with peptides comprising all or a portion of the fluorescent protein of the invention. The immunogen may comprise the complete protein, or fragments and derivatives thereof. Antibodies may be monoclonal, polyclonal, single chain or multimeric and may be produced by conventional techniques.

In another aspect the invention provides a polynucleotide which encodes a protein or polypeptide as defined above and which can be obtained by suitable modification of a AsGFP499-encoding nucleic acid molecule isolated from organisms taxonomically belonging to *Cnidaria* phylum, *Anthozoa* class, *Zoantharia* subclass, *Actinaria* order, *Anemonia* family, *Anemonia* genus and *Anemonia sulcata* species, preferably SEQ ID NO°:10 (wt-AsGFP₄₉₉ coding sequence from *Anemonia sulcata* var *rufescens).*

In a preferred embodiment, the polynucleotides according to the invention are selected from SEQ ID NOs.:11-17, a complementary strand thereof or a nucleic acid hybridising thereto under stringent conditions.

For adequate expression in mammalian cells, the nucleic acid sequences according to the invention may require optimisation of the codon usage. Preferably, the mutants according to the invention are generated from a cDNA sequence the codon usage of which has been optimised for the expression in mammalian cells, preferably from SEQ ID NO°:9, which is obtained by modifying the codon usage of SEQ ID NO°:10 (wt-AsGFP₄₉₉ coding sequence).

The fragments of a cDNA encoding a fluorescent protein according to the invention, preferably the cDNA set forth in SEQ ID NOs:11-17, as well as oligonucleotides hybridizing thereto may be used as hybridization probes against a cDNA library of a target organism of interest. Low stringency conditions may for example indicate hybridizing at 50°C and 6xSSC (0.9 M sodium chloride/ 0.09 M sodium citrate) and washing at 55°C in 1xSSC (0.15 M sodium chloride/ 0.15 M sodium citrate), whereas stringent conditions may be for example 50°C or higher and 0.1xSSC (15 mM sodium chloride/1.5 mM sodium citrate). The hybridization procedure is described in Sambrook et al.{Sambrook, 1989 #175}. If used as primers, the oligonucleotides may hybridize to different regions of the target molecule so as to amplify a desired region thereof. Depending on the length of the probe or primer, the hybridizing nucleic acid probes or primers according to the invention may have at least 90%, 95%, 97%, 98%, or at least 99% complementary with the segment of the sequence to which they hybridize. In a preferred embodiment, the oligonucleotides according to the invention contain from 10 to 30 nucleotides.

A further aspect of the invention relates to expression vectors containing the indicated nucleic acid molecules. Suitable vectors include viral and non-viral vectors, preferably plasmids, which can be used for cloning, amplifying, expressing or transferring the nucleic acid sequence into an appropriate host.

In a further aspect, the invention provides a prokaryotic or eukaryotic host cell comprising a nucleic acid of the invention or a vector thereof. Host-cells such as *E. coli, B. subtilis, S. cerevisiae,* insect cells, or cells of higher organisms such as vertebrates, particularly COS-7 cells, HEK-293, HeLa, CHO-K1, Xenopus oocytes, may be used for production of the protein or in cell-based assays.

The invention further provides transgenic organisms, the genomes of which contain a nucleic acid molecule according to the invention. Transgenic organisms can be prokaryotic or eukaryotic organisms including bacteria, cyanobacteria, fungi, plants and animals, in which one or more of the cells of the organism contain heterologous nucleic acids of the invention. The invention also includes embryonic stem (ES) cells containing a nucleic acid herein disclosed. ES cells grown on an appropriate fibroblast-feeder layer or in the presence of leukemia inhibiting factor (LIF) or other suitable growth factor(s) can be used to produce non-human transgenic animals, including non-human mammals, birds or amphibians, as previously reported. Transgenic plants may also be produced, as reviewed in Plant Biochemistry and Molecular Biology (eds. Lea and Leegood, John Wiley & Sons) (1993) pp. 275-295 and in Plant Biotechnology and Transgenic Plants (eds. Oksman-Caldentey and Barz), (2002) 719 p.

In a further aspect, the invention refers to a method for producing a fluorescent protein as above defined, which comprises the steps of:
(a) providing a nucleic acid molecule according to the invention operably linked to expression regulatory elements;
(b) introducing the nucleic acid of step (a) in a cell under conditions permissive for its expression;
(c) isolating the expression product.

Another aspect of the invention is a method for detecting fluorescence in a cell, which comprises:
(a) providing a cell expressing a fluorescent protein according to the invention;
(b) exposing the cells to excitation with light in the range of λ 460-490 nm.
(c) detecting the fluorescence.

In general, the fluorescent proteins according to the invention or their coding sequences can be used as intracellular fluorescent markers, particularly for labelling, localizing or detecting a biological molecule or a compound in a cell or cell organelle, e.g. in a cell-based assay, or they can be used for in vitro assays in a cell-free preparation, which is preferably an aqueous solution.

In typical applications, the fluorescent proteins are used as a tool for the automated screening of arrays of cells expressing fluorescent reporting groups, or as markers fused to peptides (such as targeting sequences) or proteins to detect their intracellular location, or as indicators for cellular activity e.g. in the study of signal transduction. In particular the proteins of the invention can be fused to specific domains such as the PKC-γ Ca-binding domain, PKC-γ DAG binding domain, SH2 domain or SH3 domain to detect the second messengers involved in signalling pathways{Chudakov, 2005 #106}.

In a preferred embodiment, the cell-based assays involving the fluorescent proteins of the invention are carried out in a high-throughput format utilizing an optical screening tool or apparatus suited for multi-sample analysis or by using microscopic imaging and electronic analysis.

In a further embodiment, the subject proteins are used in the screening for drug discovery and in the field of functional genomics as markers of whole cells to detect changes in multicellular reorganization and migration.

The subject fluorescent proteins also find use in protease cleavage assays as described in US 7,049,400.

In a further embodiment, the proteins of the present invention may be used in high content screening to detect co-localization of other fluorescent fusion proteins with localization markers as indicators of movements of intracellular fluorescent proteins/peptides or as markers alone.

In a further embodiment the subject fluorescent proteins are used as biosensors in prokaryotic and eucaryotic cells, such as Ca²⁺ ion indicators, pH indicators, phosphorylation indicators or indicators of other ions, such as magnesium, sodium, potassium, chloride and halides {Chudakov, 2005 #106}.

Secreted forms of the subject proteins, which can be used in a variety of different applications, can be prepared by fusing secretion leader sequences thereto.

In a preferred embodiment, the fluorescent proteins are used as *in vivo* labels (or reporter molecules) in cell and molecular biology assays, especially in assays for gene expression, protein localization and co-localization, protein-protein interactions, protein-nucleic acid interactions, nucleic acid-nucleic acid interactions, cell and cell organelle localization and interactions.

In a further preferred embodiment, the fluorescent proteins of the invention are used for detecting the effects of a test substance on the regulation of expression and/or translocation of one or more proteins of interest in a cell, or for detecting the expression of a protein of interest and the simultaneous activity of an expression control sequence in response to a test substance. The activity of two or more expression control sequences in a cell in response to a test substance may be compared by using the fluorescent proteins of the invention. Such methods may be performed in the presence and in the absence of a test substance whose effect on the process is to be measured.

In a further embodiment, the fluorescent proteins of the invention are used in fluorescence activated cell sorting (FACS) as markers of cells which can then be sorted with a fluorescent activated cell sorting device.

In addition, the subject proteins may be used in fluorescence resonance energy transfer (FRET) methods as donors and/or acceptors in combination with a second fluorescent protein or dye. Specific examples of FRET assays employing the subject fluorescent proteins include the detection of protein-protein interactions, such as in a mammalian two-hybrid system, transcription factor dimerization, membrane protein multimerization, multi-protein complex formation.

In a further embodiment, the subject fluorescent proteins are used in BRET assay (Bioluminescence Resonance Energy Transfer). BRET is a protein-protein interaction assay based on energy transfer from a bioluminescent donor to a fluorescent acceptor protein. The BRET signal is measured by the amount of light emitted by the acceptor to the amount of light emitted by the donor{Pfleger, 2006 #198}. The ratio of these two values increases as the two proteins are brought into proximity.

In a further application the fluorescent protein is used as a fluorescent timer, in which the switch of one fluorescent colour to another (e. g. green to red) concomitant with the ageing of the fluorescent protein is used to determine the activation/deactivation of gene expression.

The use of fluorescent proteins in combination with optical imaging procedures improves the knowledge of disease processes, and a more efficient evaluation of drug effects *in vivo* allows new diagnostic and therapeutic applications in human medicine. In particular the field of molecular imaging allows both visualization and quantification of molecular events associated with disease in a non-invasive and radiation-free manner using relatively simple equipment {Licha, 2005 #176} {Weissleder, 2002 #177}.

In a yet further aspect, the invention provides a kit suitable for setting up and carrying out an assay or method according to the invention. The kit typically contains a protein or a nucleic acid according to the invention, particularly the proteins of SEQ ID NOs:2-8 and/or the polynucleotides of SEQ ID NOs:11-17, or it may contain a vector and/or a preparation of cells expressing a fluorescent protein of the invention under the control of a stable or inducible promoter, and reagents suitable for running the assay. The kit may include the cDNA and the oligonucleotide primers able to produce the nucleic acid by PCR. The kit components are typically present in a suitable storage medium, such as a buffered solution, and are packaged in a suitable container. The kit may also include antibodies specific to the provided protein.

The invention will be illustrated in greater detail in the following experimental section.

### BRIEF DESCRIPTION OF THE DRAWINGS

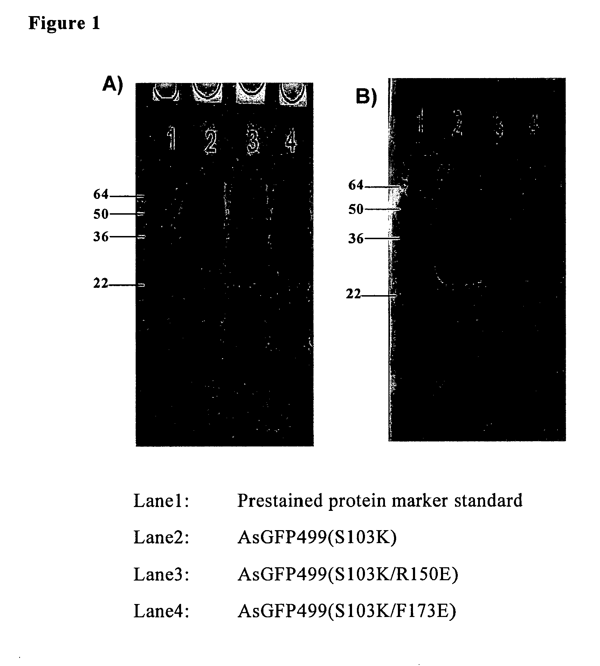

**Figure 1A****:** Coomassie stained pseudo-native SDS-PAGE gel.
**Figure 1B****:** Coomassie stained SDS-PAGE gel.

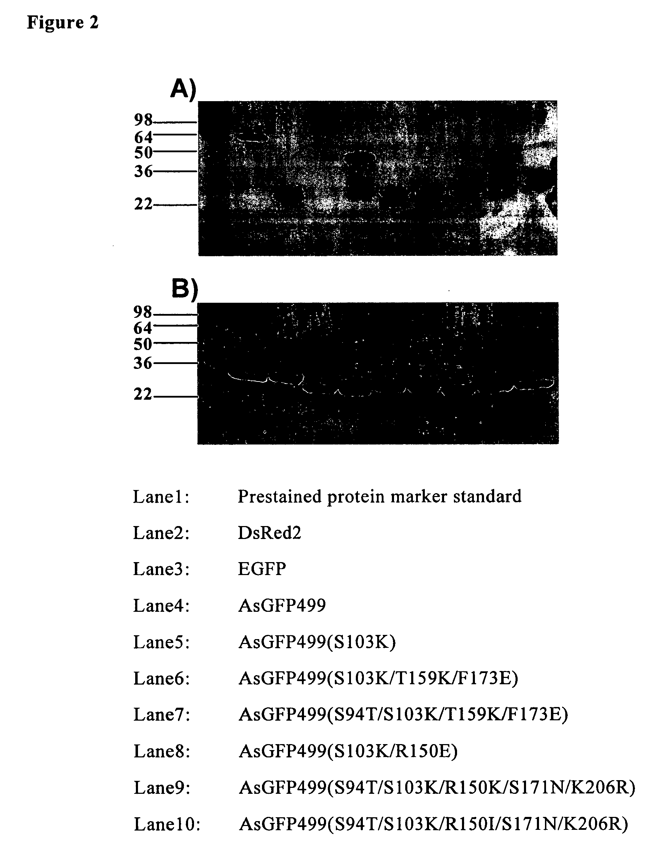

**Figure 2A**: Coomassie stained pseudo-native SDS-PAGE gel.
**Figure 2B**: Coomassie stained SDS-PAGE gel.

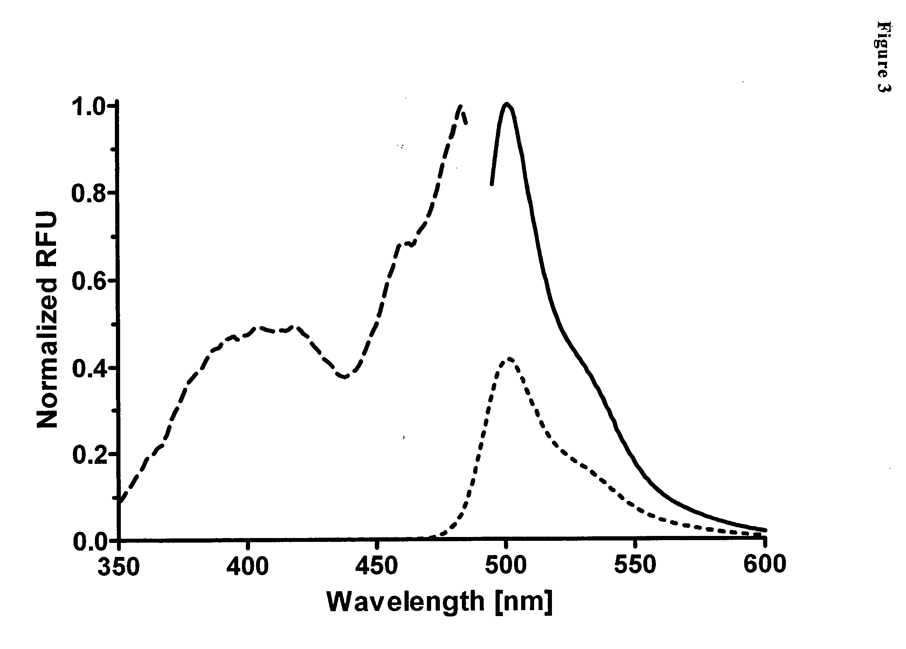

**Figure 3****:** Normalized excitation and emission spectra of wtAsGFP₄₉₉. The dashed line refers to the excitation spectra taken at fixed λ_{Em}=499 nm, the solid line to the emission spectra at λ_{Ex}=483 nm and the dotted line to the emission spectra at λ_{Em}=400 nm.

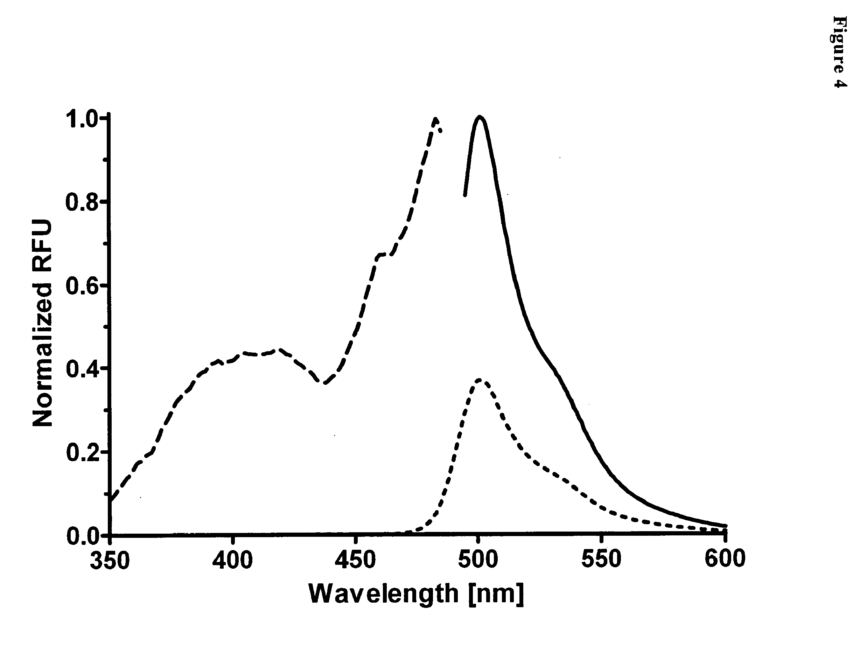

**Figure 4**: Normalized excitation and emission spectra of the mutant fluorescent protein AsGFP₄₉₉ (S103K). The dashed line refers to the excitation spectra taken at fixed λ_{Em}=499 nm, the solid line to the emission spectra at λ_{Ex}=483 nm and the dotted line to the emission spectra at λ_{Em}=400 nm.

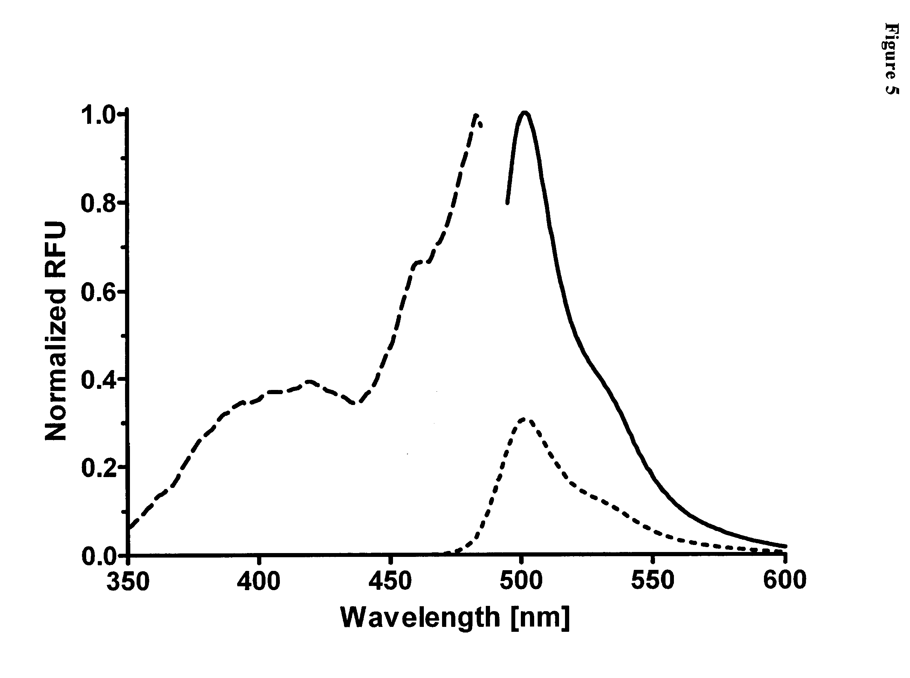

**Figure 5**: Normalized excitation and emission spectra of the mutant fluorescent protein AsGFP₄₉₉(S103K/T159K/F173E). The dashed line refers to the excitation spectra taken at fixed λ_{Em}=499 nm, the solid line to the emission spectra at λ_{Ex}=483 nm and the dotted line to the emission spectra at λ_{Em}= 400 nm.

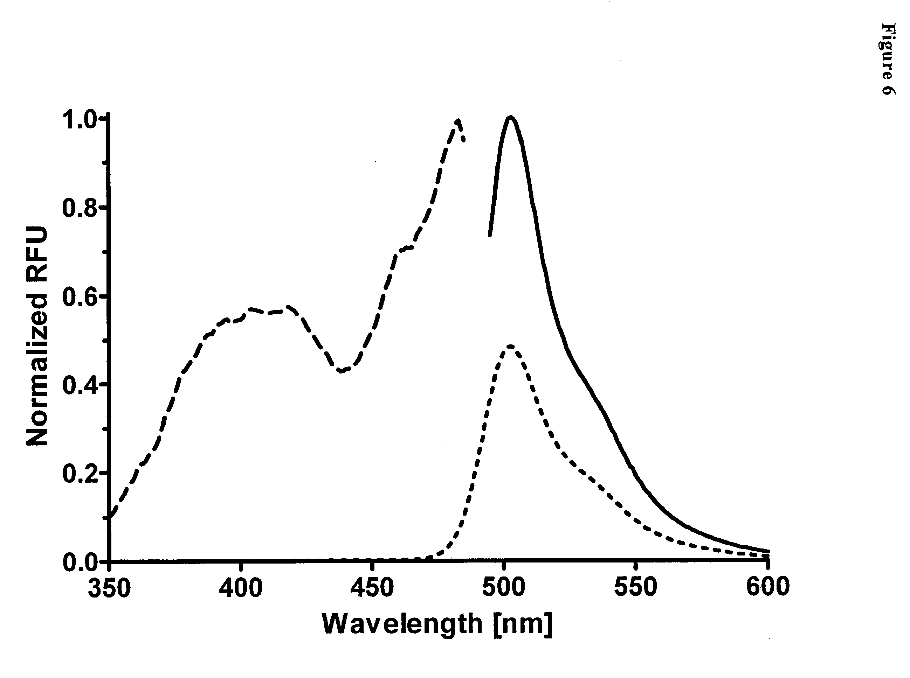

**Figure 6**: Normalized excitation and emission spectra of the mutant fluorescent protein AsGFP₄₉₉(S94T/S103K/T159K/F173E). The dashed line refers to the excitation spectra taken at fixed λ_{Em}=499 nm, the solid line to the emission spectra at λ_{Ex}=483 nm and the dotted line to the emission spectra at λ_{Em}=400 nm.

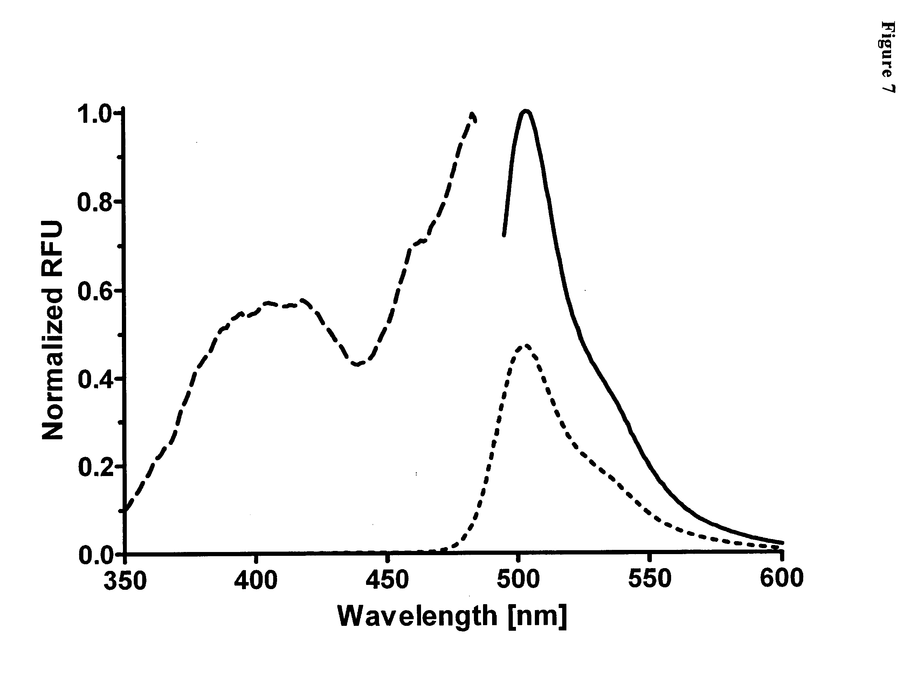

**Figure 7**: Normalized excitation and emission spectra of the mutant fluorescent protein AsGFP₄₉₉(S103K/R150E). The dashed line refers to the excitation spectra taken at fixed λ_{Em}=499 nm, the solid line to the emission spectra at λ_{Ex}=483 nm and the dotted line to the emission spectra at λ_{Em}= 400 nm.

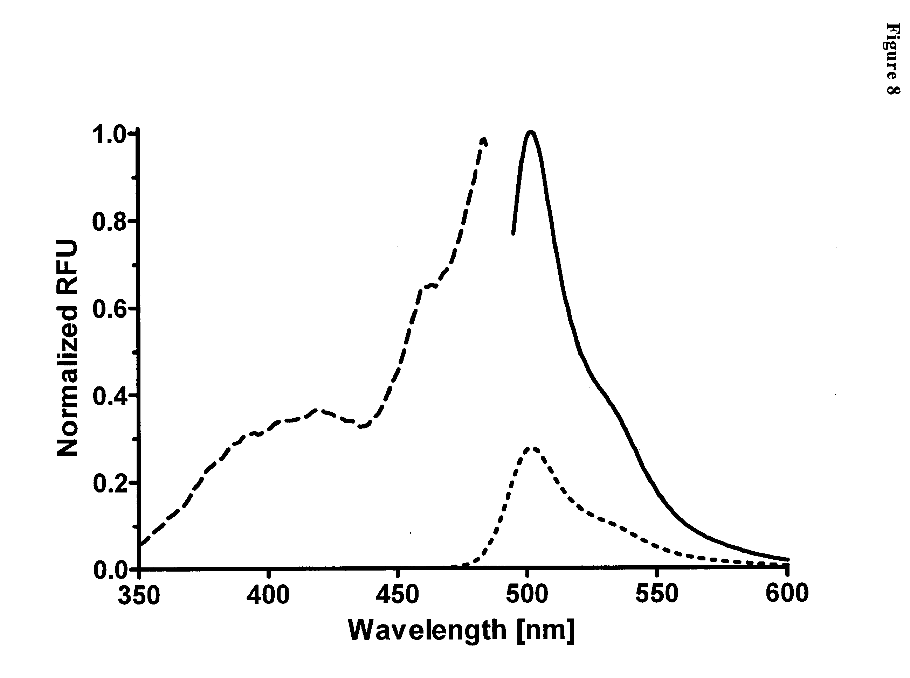

**Figure 8**: Normalized excitation and emission spectra of the mutant fluorescent protein AsGFP499(S94T/S103K/R150K/S171N/K206R). The dashed line refers to the excitation spectra taken at fixed λ_{Em}=499 nm, the solid line to the emission spectra at λ_{Ex}=483 nm and the dotted line to the emission spectra at λ_{Em}=400 nm.

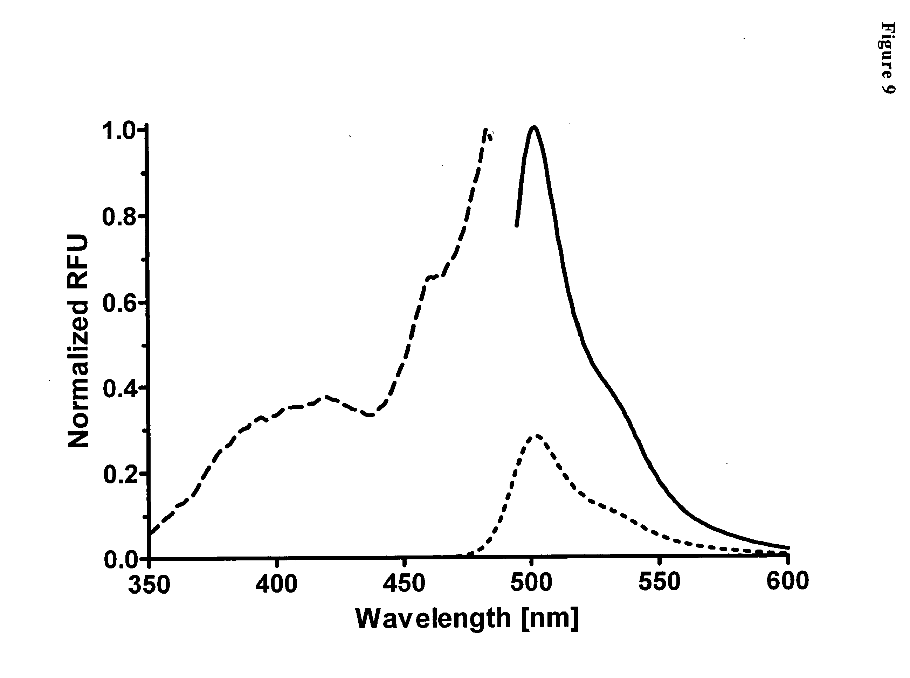

**Figure 9**: Normalized excitation and emission spectra of the mutant fluorescent protein AsGFP499(S94T/S103K/R150I/S171N/K206R). The dashed line refers to the excitation spectra taken at fixed λ_{Em}=499nm, the solid line to the emission spectra at λ_{Ex}=483nm and the dotted line to the emission spectra at λ_{Em}=400 nm.

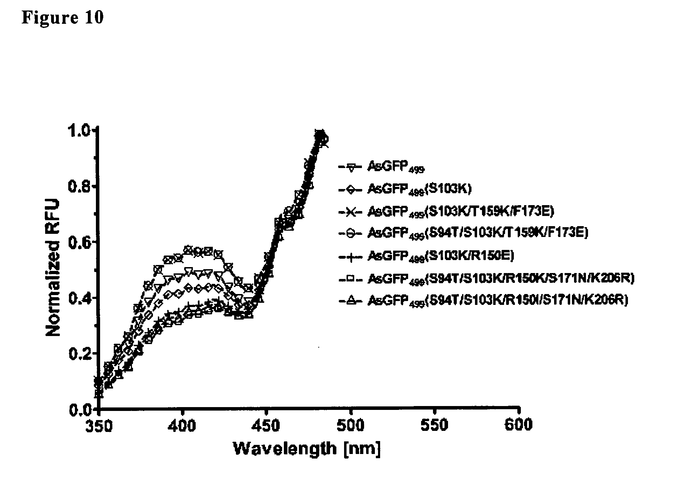

**Figure 10****:** Overlay of normalized excitation spectra of wtAsGFP₄₉₉ and its mutants.

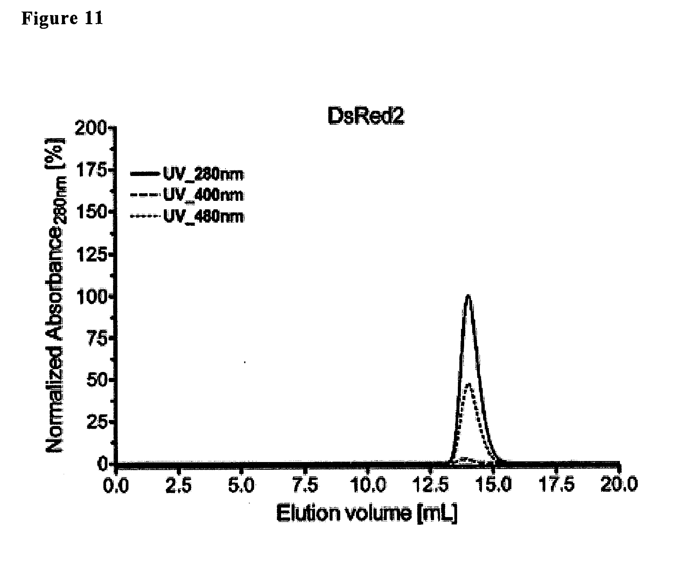

**Figure 11**: Size exclusion chromatogram of recombinant his-tagged DsRed2. The dotted line refers to the absorption at 280 nm (protein), the dashed line to 400 nm (protonated chromophore) and the solid line to 480 nm (deprotonated chromophore). The chromatogram is normalized by the peak at 280 nm.

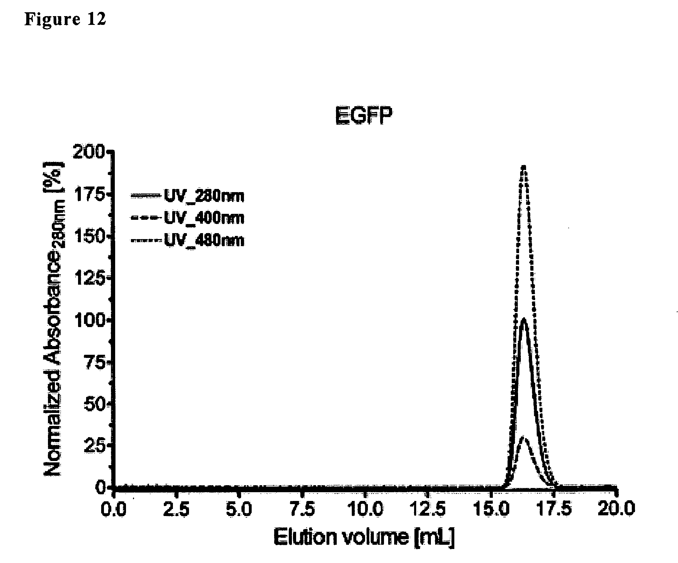

**Figure 12****:** Size exclusion chromatogram of recombinant his-tagged EGFP. The dotted line refers to the absorption at 280 nm (protein), the dashed line to 400 nm (protonated chromophore) and the solid line to 480 nm (deprotonated chromophore). The chromatogram is normalized by the peak at 280 nm.

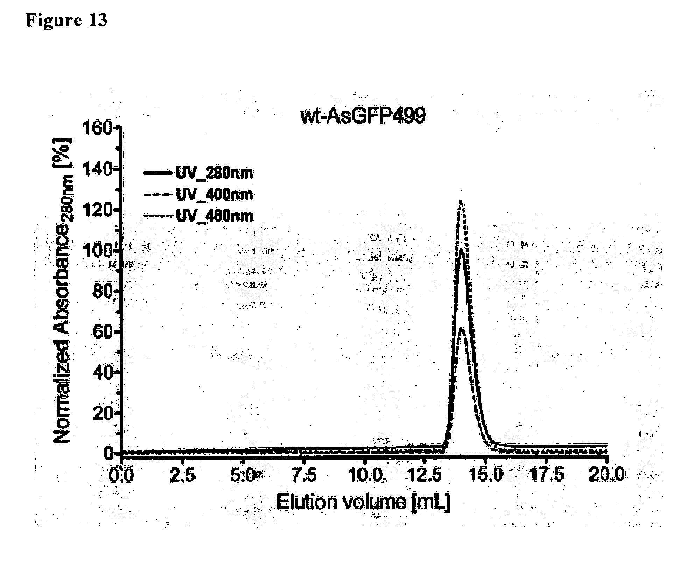

**Figure 13****:** Size exclusion chromatogram of recombinant his-tagged wt-AsGFP499. The dotted line refers to the absorption at 280 nm (protein), the dashed line to 400 nm (protonated chromophore) and the solid line to 480 nm (deprotonated chromophore). The chromatogram is normalized by the peak at 280 nm.

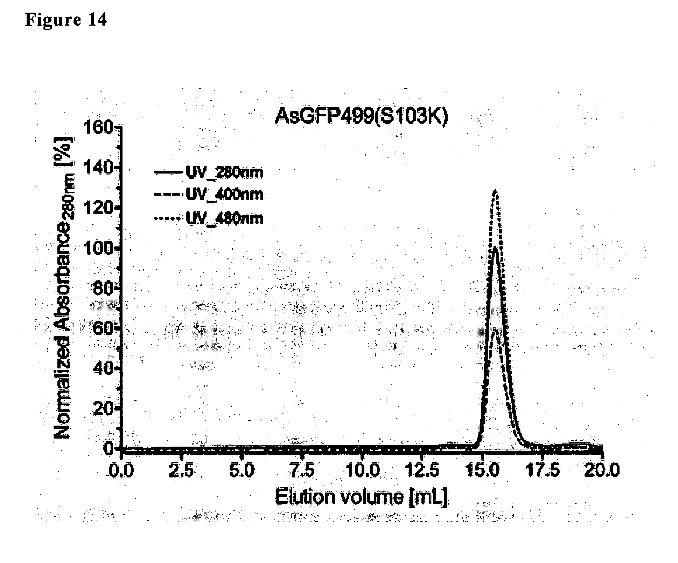

**Figure 14****:** Size exclusion chromatogram of recombinant his-tagged AsGFP499(S103K). The dotted line refers to the absorption at 280 nm (protein), the dashed line to 400 nm (protonated chromophore) and the solid line to 480 nm (deprotonated chromophore). The chromatogram is normalized by the peak at 280 nm.

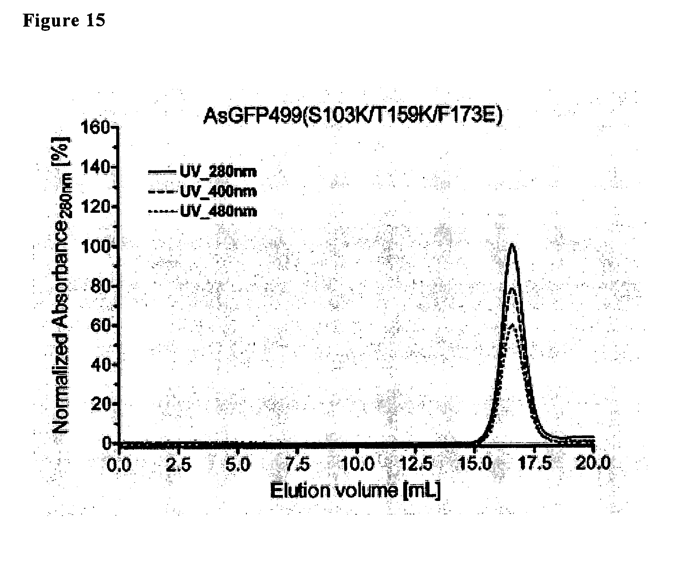

**Figure 15****:** Size exclusion chromatogram of recombinant his-tagged AsGFP499(S103K/T159K/F173E). The dotted line refers to the absorption at 280 nm (protein), the dashed line to 400 nm (protonated chromophore) and the solid line to 480 nm (deprotonated chromophore). The chromatogram is normalized by the peak at 280 nm.

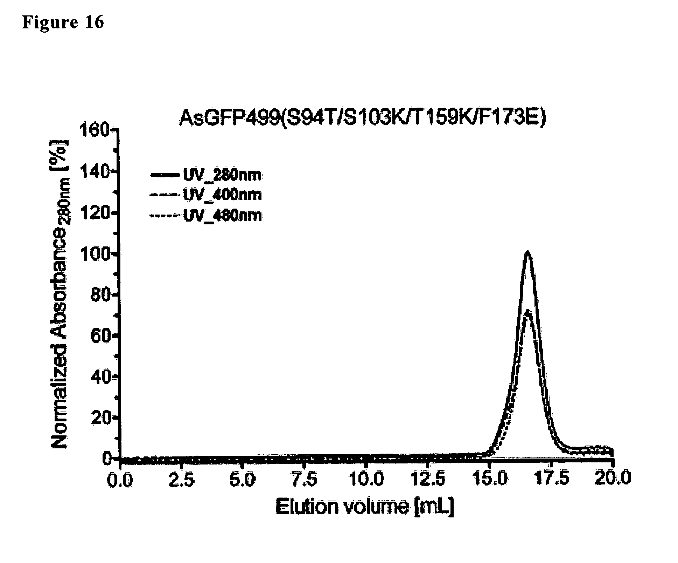

**Figure 16****:** Size exclusion chromatogram of recombinant his-tagged AsGFP499(S94T/S103K/T159K/F173E). The dotted line refers to the absorption at 280 nm (protein), the dashed line to 400 nm (protonated chromophore) and the solid line to 480 nm (deprotonated chromophore). The chromatogram is normalized by the peak at 280 nm.

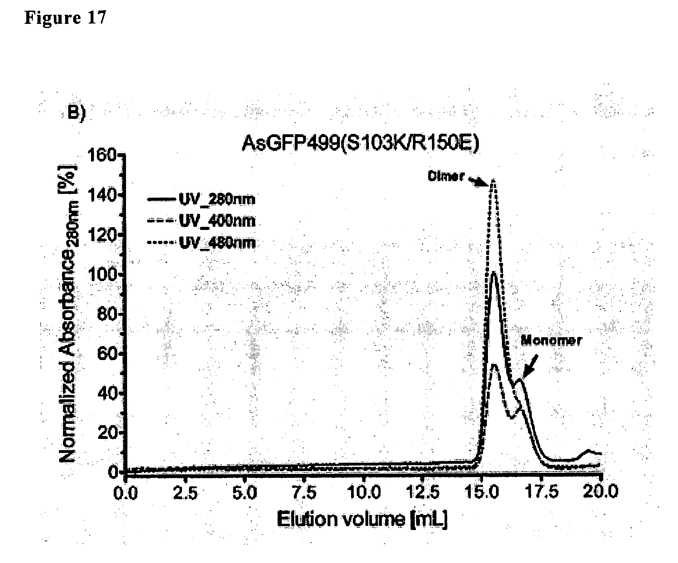

**Figure 17**: Size exclusion chromatogram of recombinant his-tagged AsGFP499(S103K/R150E). The dotted line refers to the absorption at 280 nm (protein), the dashed line to 400 nm (protonated chromophore) and the solid line to 480 nm (deprotonated chromophore). The chromatogram is normalized by the peak at 280 nm.

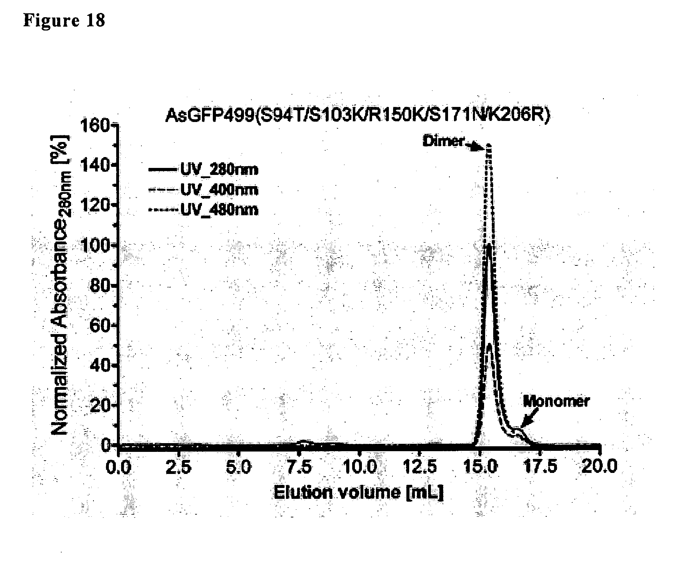

**Figure 18**: Size exclusion chromatogram of recombinant his-tagged AsGFP499(S94T/S 103K/R150K/S 171N/K206R). The dotted line refers to the absorption at 280 nm (protein), the dashed line to 400 nm (protonated chromophore) and the solid line to 480 nm (deprotonated chromophore). The chromatogram is normalized by the peak at 280 nm.

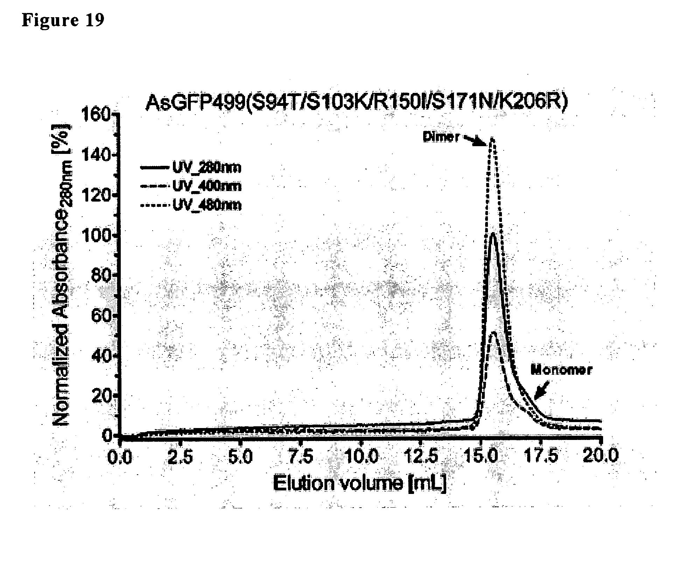

**Figure 19****:** Size exclusion chromatogram of recombinant his-tagged AsGFP499(S94T/S103K/R150I/S171N/K206R). The dotted line refers to the absorption at 280 nm (protein), the dashed line to 400 nm (protonated chromophore) and the solid line to 480 nm (deprotonated chromophore). The chromatogram is normalized by the peak at 280 nm.

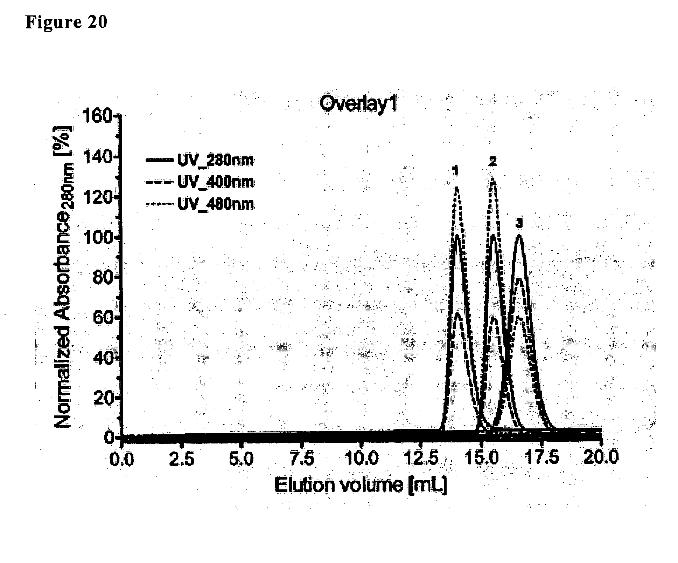

**Figure 20**: Overlaid size exclusion chromatograms of recombinant his-tagged wt-AsGFP499, AsGFP499(S103K) and AsGFP499(S103K/T159K/F173E). The dotted line refers to the absorption at 280 nm (protein), the dashed line to 400 nm (protonated chromophore) and the solid line to 480 nm (deprotonated chromophore). The chromatogram is normalized by the peak at 280 nm.

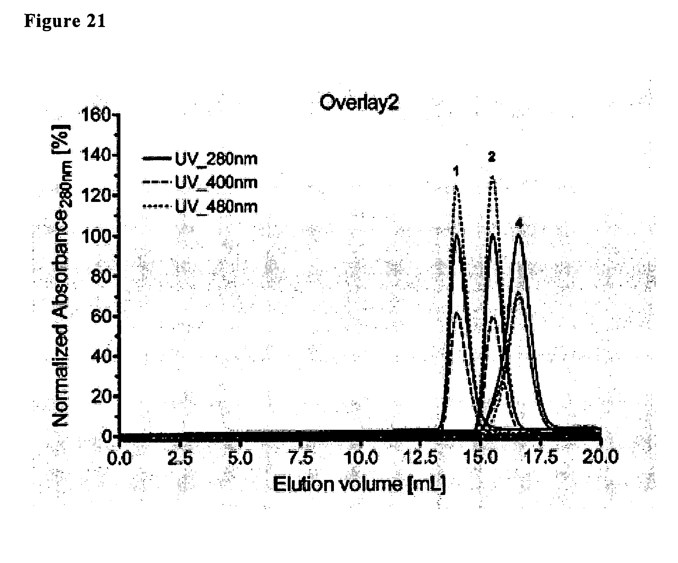

**Figure 21**: Overlaid size exclusion chromatograms of recombinant his-tagged wt-AsGFP499, AsGFP499(S103K) and AsGFP499(S94T/S103K/T159K/F173E). The dotted line refers to the absorption at 280 nm (protein), the dashed line to 400 nm (protonated chromophore) and the solid line to 480 nm (deprotonated chromophore). The chromatogram is normalized by the peak at 280 nm.

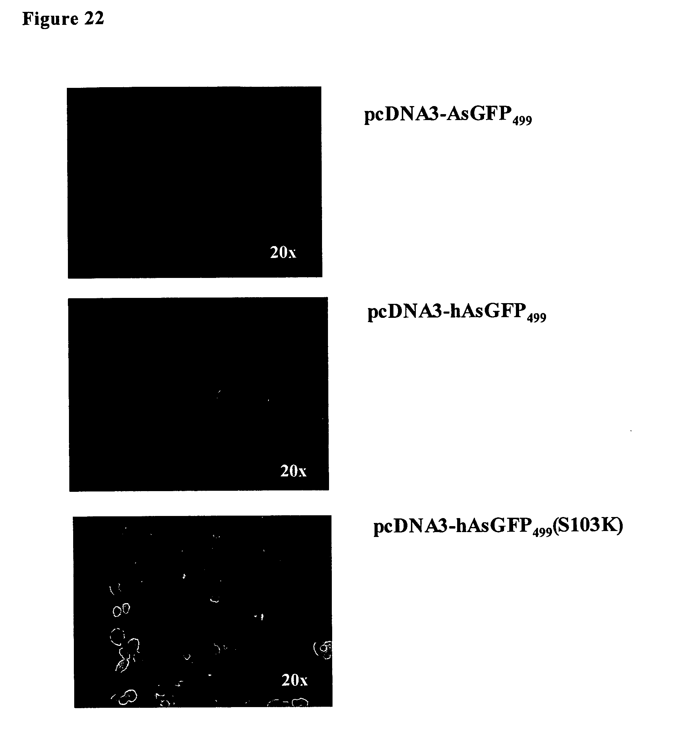

**Figure 22****:** CHO K1 cells stably transfected with the mammalian vectors pcDNA3-AsGFP499, pcDNA3-hAsGFP499 and pcDNA3-hAsGFP499(S103K).

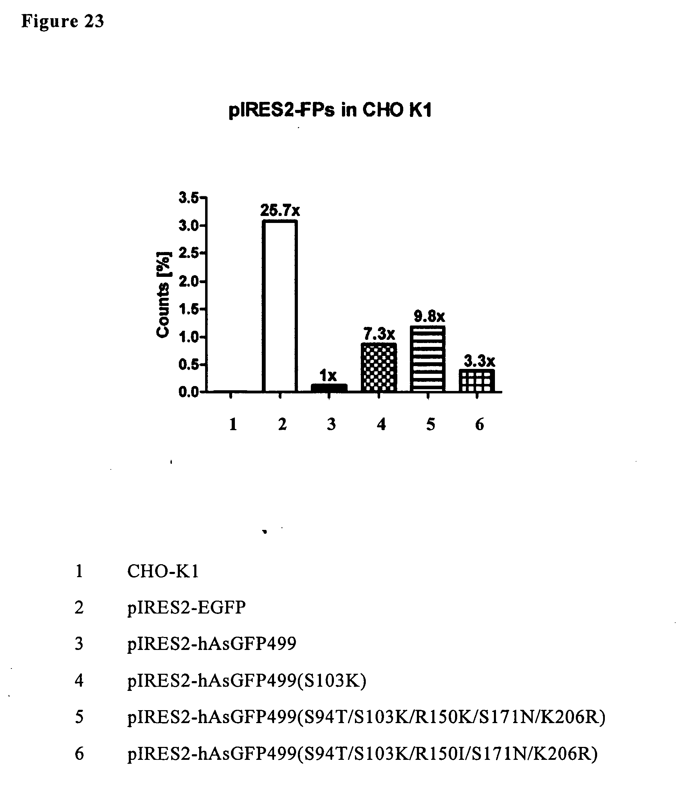

**Figure 23****:** Histogram of the amount (%) of sorted green CHO-K1 cells stably expressing the fluorescent proteins transfected with the mammalian expression vectors pIRES2-EGFP, pIRES2-hAsGFP499, pIRES2-hAsGFP499(S103K) and pIRES2-hAsGFP(S94T/S 103K/R150K/S171N/K206R) and pIRES2-hAsGFP(S94T/S103K/R150I/S171N/K206R) by FACS analysis.

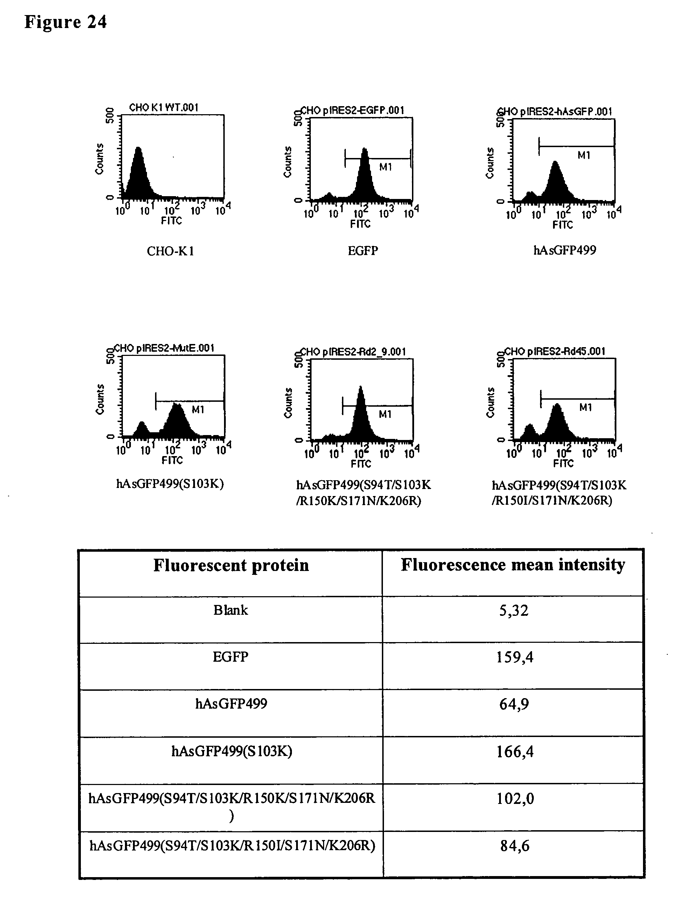

**Figure 24****:** Histograms of the fluorescence intensity of eHO-K1 cells stably transfected with the mammalian expression vectors pIRES2-EGFP, pIRES2-hAsGFP499, pIRES2-hAsGFP499(S 103K) and pIRES2-hAsGFP499(S94T/S103K/R150K/S171N/K206R) and pIRES2-hAsGFP499(S94T/S103K/R150I/S171N/K206R) by FACS. In a table are summarized the fluorescence intensities.

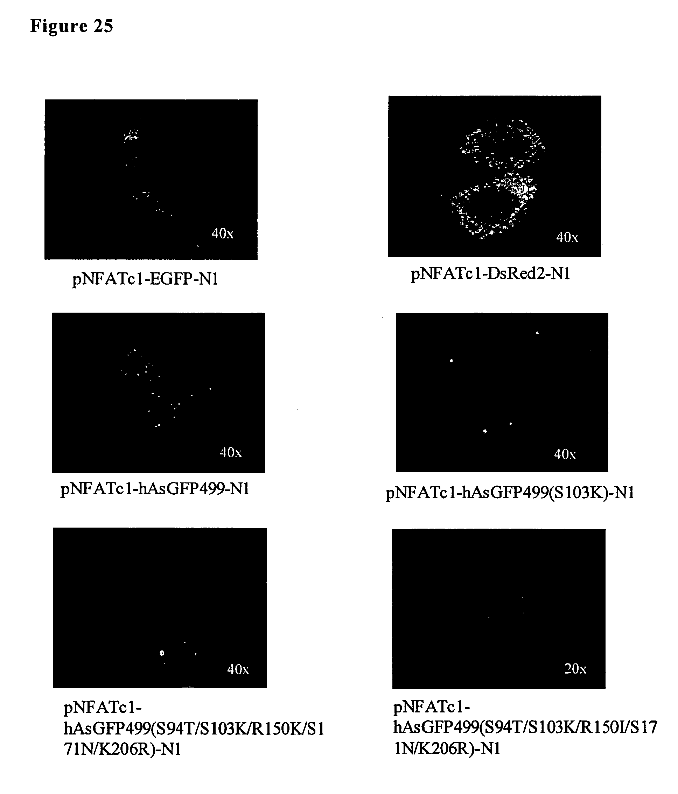

**Figure 25****:** HeLa cells transiently transfected with the mammalian fusion vectors pNFATc1-EGFP, pNFATc1-DsRed2, pNFATc1-hAsGFP499-N1, pNFATc1-hAsGFP499(S103K), pNFATc1-hAsGFP499(S94T/S103K/R150K/S171N/K206R)-N1 and pNFATc1-hAsGFP499(S94T/S 103K/R150I/S171N/K206R)-N1.

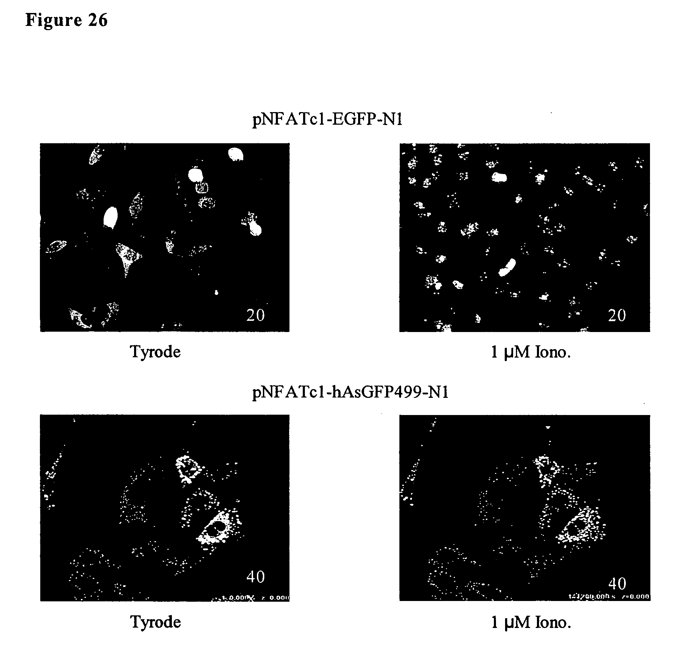

**Figure 26****:** Translocation assay in HeLa cells stably transfected with the mammalian fusion vectors pNFATc1-EGFP-N1 (images were taken with Olympus IX70 fluorescence microscope), pNFATc1-hAsGFP499-N1 after stimulation with ionomycine (images were taken with Leica confocal microscope).

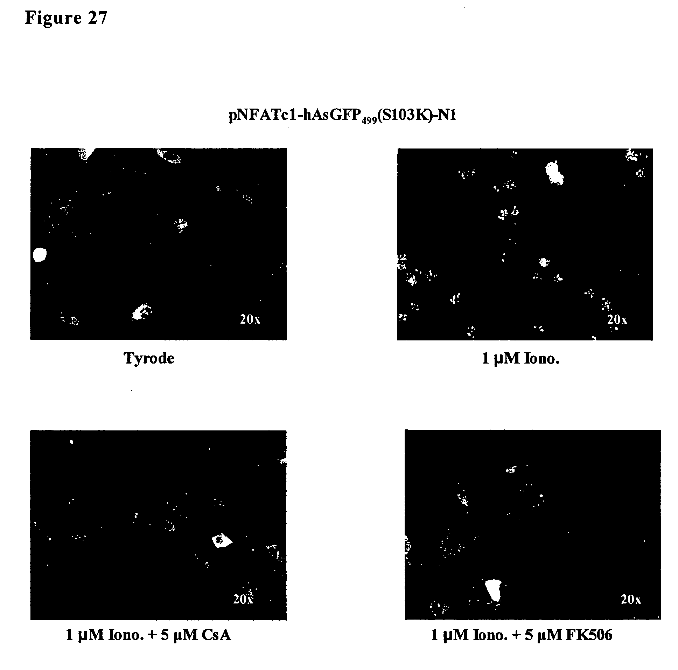

**Figure 27****:** Translocation assay in HeLa cells stably transfected with the mammalian fusion vectors pNFATc1-hAsGFP499(S103K)-N1 in presence of Calcineurin inhibitors, CsA and FK506, after stimulation with ionomycine. Images were taken with Olympus IX70 fluorescence microscope.

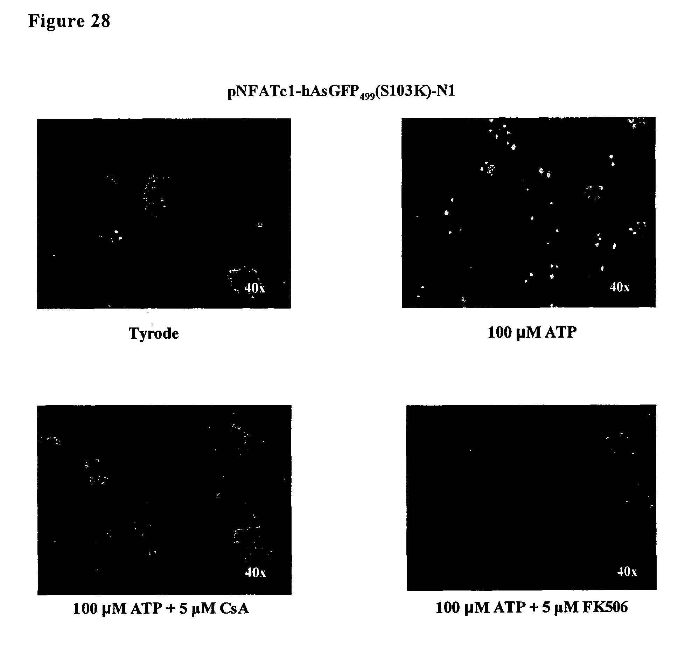

**Figure 28****:** Translocation assay in HeLa cells stably transfected with the mammalian fusion vectors pNFATc1-hAsGFP499(S103K)-N1 in presence of Calcineurin inhibitors, CsA and FK506, after stimulation with ATP. Images were taken with Olympus IX70 fluorescence microscope.

### EXAMPLE 1

### AsGFP₄₉₉ optimization for expression in mammalian cells

The codon usage of the AsGFP₄₉₉ wild-type gene wtAsGFP₄₉₉ (SEQ ID NO°:10) was adapted to the codon bias of highly expressed mammalian genes (SEQ ID NO°: 9). In addition regions of very high (> 80%) or very low (< 30%) GC content have been avoided wherever possible. For efficient translation initiation, the Kozak-consensus sequence was introduced upstream of the start codon (GENEART AG, Regensburg, Germany).

### EXAMPLE 2

### Cloning of FPs for expression in bacteria and mammalian cells

We used the following bacterial strains. Top10 (Invitrogen ) with the genotype (F⁻ *mcrA Δ*(*mrr-hsd*RMS*-mcr*BC) φ80*lac*ZΔM15 *Δlac*X74 *rec*A1 *araΔ*139 Δ(*ara-leu*)7697 *gal*U *gal*K *rpsL* (Str^{R}) *end*A1 *nup*G)*,* due to their high transformation efficiency, decreased endonuclease activity and plasmid propagation and BL21-Gold(DE3) (Stratagene) strain with the genotype *(E. coli* B F⁻ *ompT hsdS* (r_{B}⁻ m_{B}⁻) *dcm⁺* Tet^{r} *gal* λ(DE3) *endA* Hte) ideal for both, high protein expression and cloning procedures. In order to obtain competent BL21-Gold(DE3) cells with a high efficiency of transformation, we followed the standard protocol for preparing and electro-transforming BL21-Gold(DE3) cells described in the *E. coli* Pulser Transformation apparatus manual (BioRad). Transformation efficiency was tested by using pUC18 DNA and the pET DNA vectors.

**Cloning of bacterial expression vectors.** The coding sequence for EGFP, DsRed2 and hAsGFP₄₉₉ (SEQ ID NO°:9) were amplified from the vectors pEGFP-N1, pDsRed2-N1 (Clontech, USA) and pcDNA3-hAsGFP₄₉₉ with primer pairs (PRIMM, Italy) flanked by restriction sites using *Pfu* DNA polymerase (Roche, Switzerland) in the PCR reaction. The PCR products were digested with restriction enzymes (New England Biolabs) and cloned into precut pET28a(+)-vector (Novagen). The resulting vectors were transformed into electro-competent BL21(DE3) Gold bacteria. Bacteria were grown overnight at 37°C on kanamycine LB/agar. Fluorescent colonies were detected visually using an inverted fluorescence microscope (Olympus IX70).

**Cloning of mammalian expression vectors** We cloned the coding sequence of optimised wt-AsGFP499 (hAsGFP499) and its mutant AsGFP499(S103K) in the mammalian expression vector pcDNA3 (Invitrogen), to compare the fluorescence intensity in cells, with the not optimised version of wt-AsGFP499. We substituted EGFP in the pEGFP-N1 fusion vector with the coding sequences of hAsGFP₄₉₉ and its mutants using BamHI and NotI restriction sites. To investigate the shuttling behaviour of NFATc1 in fusion with fluorescent proteins (FPs), we inserted the coding sequence for NFATc1 in frame to the FPs, (pcDNA3-NFATc1){Chin, 1998 #146}. Suitability of hAsGFP499 and its mutants as selection markers for FACS was tested by substitution of EGFP in the pIRES2-EGFP vector with the coding sequences of hAsGFP₄₉₉ and its mutants. The resulting vectors were transformed into chemical competent Top10 bacteria. Bacteria were grown overnight at 37°C on appropriate antibiotic LB/agar plates.

For plasmid production, single colonies were grown o/n at 37°C by shaking with 220 rpm in 3mL LB-medium containing the appropriate antibiotics. After recovery of the bacterial pellets, the plasmids were extracted using a Mini-prep Kit (QIAGEN). The accuracy of the cloned products was either checked by digestions of the DNA using restriction enzymes or by submitting for sequencing (PRIMM).

Primer pairs (oligonucleotides) that we used here for cloning are listed in **Table 1.**

**Table 1**

| Oligonucleotides used for cloning of AsGFP₄₉₉ (SEQ ID NO°:9) in target vectors: | |
|---|---|
| pET28a(+)-Fw.: | 5'-CGGCAGC**CATATG**TACCCCAGCATCAAG-3' |
| | **NdeI-restriction site,** (SEQ ID NO :18) |
| pET28a(+)-Fw.: | 5'-GCG**GATCC**TTATCAATTGTGGCCCAG-3' |
| | **BamHI-restriction site**, (SEQ ID NO :19) |
| pRSETB-Fw.: | 5'-TAA**GGATCC**GATGTACCCCAGCATC-3' |
| | **BamHI-resriction site**, (SEQ ID NO :20) |
| pRSETB-Rev.: | 5'-CAT**GGTACC**TCAATTGTGGCCCAG-3' |
| | **KpnI-resriction site**, (SEQ ID NO :21) |
| pcDNA3-Fw.: | 5'-CTT**GGTACC**ATGTACCCCAGCATC-3' |
| | **KpnI-resriction site**, (SEQ ID NO :22) |
| pcDNA3-Rev.: | 5'-TAGAGTC**GCGGCCGC**TCAATTGTGcCCCAGCTTGC-3' |
| | **NotI-resriction site; c instead of G (Hairpin)** (SEQ ID NO :23) |
| phAsGFP-Fw.: | 5'-CCG**GGATCC**ACCGGTCGCCACCATGTACCCCAGCATCAA |
| | G-3' **BamHI-resriction site**, (SEQ ID NO :24) |
| phAsGFP-Rev.: | 5'-TAGAGTC**GCGGCCGC**TCAATTGTGcCCCAGCTTGC-3' |
| | **NotI-resriction site; c instead of G (Hairpin)** (SEQ ID NO :25) |
| pIRES2-Fw.: | 5'-TACCCCAGCATCAAGGAGACC-3' |
| | hAsGFP₄₉₉ without startcodon (ATG) (SEQ ID NO :26) |
| pIRES2-Rev.: | 5'-TAGAGTC**GCGGCCGC**TCAATTGTGcCCCAGCTTGC-3' |
| | **NotI-resriction site; c instead of G (Hairpin)** (SEQ ID NO :27) |
| Oligonucleotides | used for cloning of NFATc1 in phAsGFP₄₉₉-N1 vector |
| NFATc1-Fw.: | 5'-GA**GGTACC**ATGCCAAGCACCAGCTTTC-3' |
| | **KpnI-resriction site**, (SEQ ID NO :28) |
| NFATc1-Rev.: | 5'-CT**GGATCC**cgGAAAAAGCACCCCACGC-3' |
| | Stopcodon of NFATc1 is substituted with **GGATCC**cg |
| | **BamHI-resriction site**, (SEQ ID NO:29) |

### EXAMPLE 3

### Mutagenesis

Site directed mutagenesis (SDM) technique is a controlled process that deliberately creates specific mutations in a gene in order to study the effects on the associated protein However, site directed mutagenesis becomes challenging when the crystal structure of the protein of interest, in our case the structure of AsGFP₄₉₉, was unknown. Since GFP-like proteins are believed to share the same β-can structure, we used existing data, sequences and crystal structures of other fluorescent proteins and chromoproteins of the GFP-family, to predict crucial residues for oligomerization of AsGFP₄₉₉. SDM was performed using the QuikChange® Site-Directed Mutagenesis Kit (Stratagene) following their instructions Briefly summarized, the vector pET28a(+) containing the mammalian version of AsGFP₄₉₉ or its mutants was fully synthesized with matching mutagenic primer pairs using Pfu Turbo DNA polymerase by PCR (Roche ,Switzerland). The parental templates were eliminated with DpnI that selectively digests only methylated and hemi-methylated DNA. The resulting nicked PCR vectors, containing the desired mutation, were subsequently transformed into electro-competent BL21(DE3) Gold bacteria. All mutations were confirmed by sequencing.

Since pET28a(+) is not an ideal vector for random mutagenesis, the vector pRSETB (Stratagene) was used. The mutants AsGFP₄₉₉(S 103K/R150E) and AsGFP₄₉₉(S103K/T159K/F173E), previously generated by SDM were recloned between the restriction sites BamHI/KpnI using the primer pair (SEQ ID NO :20 and 21) given in **Table 1.** Error-prone PCRs were done with the Genemorph Kit II (Stratagene) {Miyazaki, 2003 #154} using the same primer pair as above. The transformed BL21(DE3) Gold bacteria with nicked PCR vectors were grown overnight on ampicilline LB/agar plates at 37°C. Interesting mutants were recloned into the pET28a(+) vector.

Mutagenic primer pairs that we used for SDM are listed in **Table 2.**

**Table 2**

| Oligonucleotides used for site-directed-mutagenesis (bold letters indicate the mutation: | |
|---|---|
| S94T -Fw.: | 5'-GGGAGAGAGTG**ACC**ACCTACGAGGAC-3' (SEQ ID NO :30) |
| S94T -Rev.: | 5'-GTCCTCGTAGGTGGTCACTCTCTCCC-3' (SEQ ID NO :31) |
| E97G-Fw.: | 5'-GAGTGAGCACCTAC**GGC**GACGGCGGCGTG-3' (SEQ ID NO :32) |
| E97G-Rev.: | 5'-CACGCCGCCGTC**GCC**GTAGGTGCTCACTC-3' (SEQ ID NO :33) |
| S 103K-Fw.: | 5'-GGCGGCGTGCTG**AAG**GCCACCCAGGAG-3' (SEQ ID NO :34) |
| S103K -Rev.: | 5'-CTCCTGGGTGGC**CTT**CAGCACGCCGCC-3' (SEQ ID NO :35) |
| R150E-Fw.: | 5'- ACCGTGATCCCC**GAG**GATGGCGGCCTG-3' (SEQ ID NO :36) |
| R150E-Rev.: | 5'-CAGGCCGCCATC**CTC**GGGGATCACGGT-3' (SEQ ID NO :37) |
| R157G-Fw.: | 5'-GCCTGCTGCTG**GGC**GACACCCCTGCC-3' (SEQ ID NO :38) |
| R157G-Rev.: | 5'-GGCAGGGGTGTC**GCC**CAGCAGCAGGC-3' (SEQ ID NO :39) |
| T159K-Fw.: | 5'-TGCTGCTTAGAGAC**AAA**CCTGCCCTCATGC-3' (SEQ ID NO :40) |
| T 159K-Rev.: | 5'-GCATGAGGGCAGG**TTT**GTCTCTAAGCAGCA-3' (SEQ ID NO :41) |
| T 159A-Fw.: | 5'-TGCTGCTTAGAGAC**GCA**CCTGCCCTCATGC-3' (SEQ ID NO :42) |
| T159A-Rev.: | 5'-GCATGAGGGCAGG**TGC**GTCTCTAAGCAGCA-3' (SEQ ID NO :43) |
| S171A-Fw.: | 5'-CGGCGGCCACCTa**GCA**TGCTTTATGGAGAC-3' (SEQ ID NO :44) |
| S171A-Rev.: | 5'-GTCTCCATAAAGCA**TGC**tAGGTGGCCGCCG-3' (SEQ ID NO :45) |
| F 173H-Fw.: | 5'-CACCTGAGCTGC**CAC**ATGGAGACCACC-3' (SEQ ID NO :46) |
| F 173H-Rev.: | 5'-GGTGGTCTCCAT**GTG**GCAGCTCAGGTG-3' (SEQ ID NO :47) |
| F173A-Fw.: | 5'-CACCTGAGCTGC**GCC**ATGGAGACCACC-3' (SEQ ID NO :48) |
| F173A-Rev.: | 5'-GGTGGTCTCCAT**GGC**GCAGCTCAGGTG-3' (SEQ ID NO :49) |
| F173E-Fw.: | 5'-CACCTGAGCTGCGAGATGGAGACCACC-3' (SEQ ID NO :50) |
| F 173E-Rev.: | 5'-GGTGGTCTCCATCTCGCAGCTCAGGTG-3' (SEQ ID NO :51) |

### EXAMPLE 4

### Protein production and purification

Fluorescent colonies were picked and grown overnight on a shaker at 37°C in 2 mL liquid LB medium containing the appropriate antibiotic. 5 mL fresh medium was inoculated with the overnight bacterial cultures and grown to A₆₀₀ 0.6-0.8. The FP expression was induced at 23, 30 or 37°C (the choice of the temperature was depending on the mutant) by addition of IPTG to 0.5 mM. After overnight incubation of the cells, small aliquots (50 uL) of the suspension were transferred into 96 well plates (MTP) for fluorescence measurements (Safire2, Tecan) to ascertain proper protein production and/or spectroscopically shifted variants. 4 mL of the cultures were centrifuged and the pellets were then lysed in 200µL BPER-II (Pierce) solution added with 30 mM Tris-HCl pH8, 300mM NaCl, 10µg/mL DNAse and EDTA-free protease-inhibitors (Boehringer Mannheim, Germany). Finally the bacterial lysates were centrifuged and the supernatants were analyzed on pseudo-native SDS-PAGE.{Baird, 2000 #35}.

For large scale protein purification, 200mL LB medium containing kanamycine were inocculated with an overnight culture of fluorescent bacteria and grown at 37°C until OD₆₀₀ₙₘ:0.6-0.8 was reached. Protein production was induced with 500 µM IPTG. Samples were lysed and centrifuged as described, subsequent protein purifications were performed on ÄKTA explorer 100 (GE Healthcare). The supernatants were applied on 1mL His-Trap columns (GE Healthcare). The columns containing the his-tagged proteins bound to stationary Ni²⁺ ions were washed in a multistep procedure with a 10 mM Tris-HCl+150 mM NaCl pH8 buffer containing varying imidazole concentrations (10-400 mM). After protein elution, the imidazol buffer was exchanged against 10 mM Tris-HCl+150mM NaCl pH7.4 using Amicon 10 filter units. The protein concentrations were determined using the BCA Assay Kit (Pierce) and further used in biochemical characterizations.

### EXAMPLE 5

### SDS-PAGE

Heated (SDS-PAGE) or not heated (pseudo-native SDS-PAGE) protein samples mixed 1:5 with 5x SDS sample buffer containing 200 mM DTT were loaded on 16% Tris-Glycine gels (Invitrogen) and separated in 1x Tris-Glycine-SDS buffer at pH 8. As protein weight markers were used prestained SeaBlue plus2 (Invitrogen) and (His₆)-tagged DsRed2 (tetramer) and EGFP (monomer). Following separation, gels were illuminated with UV light to ascertain fluorescent bands and later stained in Coomassie blue. The stained gels are shown in **Figures 1** **and** **2****.**

### Results and conclusions

The wt AsGFP499 migrates as a tetramer with a MW of >64 kDa (**Fig. 2A** **Lane 4).** On the contrary, AsGFP499(S103K) migrates as a dimer with a MW of ≤50 kDa and shows a faint protein smear towards lower molecular weights **(****Fig. 2A** **Lane 5).** The mutants AsGFP499 (S103K/T159K/F173E), AsGFP499 (S94T/S103K/T159K/F173E) and AsGFP499 (S94T/S103K/R150I/S171N/K206R) run comparable with monomeric EGFP (**Fig. 2A** **Lane 3; 6; 7 and 10**) in agreement with a projected theoretical MW of ≈28 kDa for a monomeric protein, confirming the mutants to be monomeric variants. AsGFP499 (S103/R150E) and AsGFP499 (S103/F173E) show partial monomeric character **(****Fig. 2A** **Lane 8 and** **Fig. 1A** **Lane 4).** The band at the height of a dimer was clearly fluorescent under UV irradiation. For the random mutant AsGFP499(S94T/S103K/R150K/S171N/K206R) a sharp band cannot be observed (**Fig. 2A** **Lane 9**). A protein smear is visible from the height of a dimer to the height of a monomer, thus indicating a partial monomeric character.

When the protein samples were heated before loading onto the gel, no fluorescent bands were visible. After staining with Coomassie-blue, monomeric protein bands are detectable at a MW of >22 kDa **(****Fig. 1B** **and** **2B****).**

### EXAMPLE 6

### Spectroscopic studies

Fluorescence spectra were taken with TECAN Safire2 spectrofluorimeter at room temperature. Excitation scans in a range from 350 nm to 490 nm were measured with a fixed emission wavelength at 499 nm and emission scans in a range from 450 nm to 600 nm with a fixed excitation wavelength at 400 and 483 nm.

### Results and conclusions

The excitation spectra of all fluorescent proteins show two λₘₐₓ. **(****Fig. 3-9****)** The first peak is ~400nm and the second one ~483nm. The excitation peak at 400 nm is the result of the protonated state of the chromophoric group and the peak at 483 the deprotonated one{Nienhaus, 2006 #170}. In comparison to the wild type protein, the mutants show improved spectral properties. They favour the deprotonated state. The monomeric mutant AsGFP₄₉₉(S103K+T159K+F173E) seems to favour both transition states (**Fig. 10****).**

### EXAMPLE 7

### Size exclusion chromatography (SEC)

The apparent molecular weights of the purified (His₆)-tagged FPs were determined using SEC on a pre-calibrated Superdex 200HR10/30 column (GE Healthcare) connected to ÄKTAexplorer 100 (GE Healthcare). The absorbance was recorded simultaneously at three wavelengths (280, 400 and 480) using the UV-900 Detector. By a flow rate of 0.5 mL/min, 100 µL of the purified protein solutions (2 mg/mL) were separated in a 10 mM Tris buffer containing 200 mM NaCl at pH 8.

### Results and conclusions

SEC experiments showed that all filtered mutants and EGFP are smaller in size than the wild type protein and DsRed2 (**Fig. 11-21**), thus exhibit dimeric and/or monomeric characteristics. The mutants AsGFP499 (S103/R150E) and AsGFP499 (S94T/S103K/R150K/S171N/K206R) that show dimeric and monomeric bands or a protein smear on pseudo-native SDS-PAGE, are separated here and seem to exist as partial monomers. Surprisingly AsGFP499 (S94T/S103K/R150I/S171N/K206R) that migrated on pseudogel as a monomer, shows a similar elution profile which indicates also here the existence of dimers and monomers (**Fig. 17-19**). It is likely that at a certain protein concentrations, these proteins tend to dimerize as it was reported for the wild type GFP from Aequorea victoria.

The elution volumes and calculated apparent molecular weight are summarized in the following Table 3:

**Table 3**

| **Recombinant his-tagged protein** | **Elution volume [mL]** | **Apparent MW [kDa]** |
|---|---|---|
| DsRed2 | 14,02 | ≈79 |
| EGFP | 16,.31 | ≈27 |
| wt-AsGFP499 | 14,05 | ≈78 |
| AsGFP499(S103K) | 15.54 | =39 |
| AsGFP499(S103K/T159K/F173E) | 16,62 | ≈23 |
| AsGFP499(S94T/S103K/T159K/F173E) | 16.60 | = 23 |
| AsGFP499(S103K/R150E) | 15,55 and 16,62 | ≈ 39 and 23 |
| AsGFP499(S94T/S103K/R150K/S171N/K206R) | 15,53 and 16,61 | ≈39 and 23 |
| AsGFP499(S94T/S103K/R150I/S171N/K206R) | 15,56 and 16,63 | ≈39 and 23 |

### EXAMPLE 8

### Transfection of mammalian cells

CHO-K1 cells were used for the expression of FPs cloned either into pcDNA3 or pIRES2 vector. Cells were transfected using FuGENE^{®} 6 Reagent (Roche). 8 hours after the transfection, transiently expressed fluorescent proteins in cells were observed through an inverted microscope IX70 (Olympus). Cells stably expressing the fluorescent proteins were sorted by FACS (Becton and Dickinson) and maintained in culture for at least 3 months.

HeLa cells were used for the expression of NFATc1 cloned into fusion vectors containing hAsGFP499 or its mutants. Cells were transfected as mentioned above. 24 hours later, transiently expressed fusion-proteins in cells could be observed through an inverted microscope IX70 (Olympus). Antibiotic resistant clones were selected with 2 mg/ml and maintained in 0.8 mg/mL Gentamycin (G418). Cells stably expressing the fusion proteins were one day before treated with a histone-deacetylase inhibitor TSA (TrichostatinA, Sigma-Aldrich), in order to increase the gene-expression and used for FACS sorting.

### Results and conclusions:

The CHO-K1 cells stably transfected with the pcDNA3-AsGFP499(S103K) seem to be more fluorescent than wt-AsGFP499 and hAsGFP499 (**Fig. 22**). Obviously the mammalian version (hAsGFP499) is highly expressed in cells than the wild type one (wt-AsGFP499). But if we compare the mutant (hAsGFP499(S 103K)) with the mammalian version then it seems that the mutant is more fluorescent. We assume that either it could be more expressed, more soluble or its quantum yield could be higher than hAsGFP499.

The FACS sorting results **(****Fig. 23** **and** **24****)** are indicating that the mutants are more suitable as selection markers compared to hAsGFP499. CHO K1 cells stably expressing the mammalian vectors pIRES2-hAsGFP (S103K), pIRES2-hAsGFP (S94T/S 103K/R150K/S 171N/K206R) and pIRES2-hAsGFP (S94T/S103K/R150I/S171N/K206R) show that the amount of sorted cells are 3-10 times higher than in cells expressing the hAsGFP499 protein. And if we compare their fluorescence intensity, the mutants seem to be more fluorescent than hAsGFP499 (**Fig. 24**).

HeLa cells transiently expressing the fusion protein NFATc1-DsRed2 and NFATc1-AsGFP499 show aggregation in cytoplasm. (Fig. 25). On the contrary, the mutants show a similar pattern of distribution as EGFP. In these cells the fusion proteins are homogenously distributed in the cytoplasm. As it was reported before, oligomeric fluorescent proteins in fusion to target proteins could alter the physiological localization of them. Although our mutants show dimeric and partial monomeric character, the localization of NFATc1 seems not to be influenced by them.

### EXAMPLE 9

### Translocation assay of NFATc1 in fusion with FPs

On day one, HeLa cells stably expressing the fusion proteins were treated with TSA. The following day the cells were stimulated either with ionomycine (Sigma-Aldrich) or ATP (Sigma-Aldrich) in presence or absence of Calcineurine (CaN) inhibitors Ciclosporine A (CsA) (Sigma-Aldrich) or FK506. (Sigma-Aldrich). We monitored the NFATc1 translocation using a confocal microscope or we used an inverted fluorescence microscope IX70 (Olympus) to take images.

Increased intracellular levels of calcium-ions stimulate the translocation of NFATc1{Rao, 1997 #50} from the cytoplasm into the nucleus. As a positive control we used EGFP in fusion with NFATc1. As in transient expressions (**Fig. 25**), the stably expressed fusion protein is well distributed in the cytoplasm. After stimulation with 1 µM ionomycine, the fusion product translocates into the nucleus (**Fig. 26**). This process takes more or less 20 min. But cells expressing the fusion protein with hAsGFP₄₉₉, show aggregates. No translocation is visible after stimulation with 1µM ionomycine **(****Fig. 26****).**

HeLa cells stably expressing the fusion protein NFATc1-hAsGFP499(S 103K) show a homogenous distribution in the cytoplasm. The stimulation with 1 µM ionomycine or 100 µM ATP lead to a translocation of NFATc1 into the nucleus. To test whether the translocation occurs due to NFATc1, we incubated the cells one hour earlier with 5µM of the inhibitors CsA or FK506 and then treated the cells again with 1µM ionomycine or 100 µM ATP. Both inhibitors completely blocked the translocation of the fusion protein even in presence of the stimulators(**Fig. 27** **and** **28**).

### Reagents

All other chemicals were from standard sources and were of reagent grade or better.

### REFERENCES

1. Wiedenmann, J.; Elke, C.; Spindler, K. D.; Funke, W. Cracks in the beta-can: fluorescent proteins from Anemonia sulcata (Anthozoa, Actinaria). Proc Natl Acad Sci U S A 2000, 97, 14091-6.
2. Nienhaus, K.; Renzi, F.; Vallone, B.; Wiedenmann, J.; Nienhaus, G. U. Chromophore-protein interactions in the anthozoan green fluorescent protein asFP499. Biophys J 2006, 91, 4210-20.
3. Johnson, F. H.; Gershman, L. C.; Waters, J. R.; Reynolds, G. T.; Saiga, Y.; Shimomura, O. Quantum efficiency of Cypridina luminescence, with a note that of Aequorea. J Cell Comp Physiol 1962, 60, 85-104.
4. Morin, J. G.; Hastings, J. W. Energy transfer in a bioluminescent system. J Cell Physiol 1971, 77, 313-8.
5. Wampler, J. E.; Hori, K.; Lee, J. W.; Cormier, M. J. Structured bioluminescence. Two emitters during both the in vitro and the in vivo bioluminescence of the sea pansy, Renilla. Biochemistry 1971, 10, 2903-9.
6. Morin, J. G.; Reynolds, G. T. The cellular origin of bioluminescence in the colonial hydroid Obelia. Biol Bull 1974, 147, 397-410.
7. Labas, Y. A.; Gurskaya, N. G.; Yanushevich, Y. G.; Fradkov, A. F.; Lukyanov, K. A.; Lukyanov, S. A.; Matz, M. V. Diversity and evolution of the green fluorescent protein family. Proc Natl Acad Sci U S A 2002, 99, 4256-61.
8. Matz, M. V.; Fradkov, A. F.; Labas, Y. A.; Savitsky, A. P.; Zaraisky, A. G.; Markelov, M. L.; Lukyanov, S. A. Fluorescent proteins from nonbioluminescent Anthozoa species. Nat Biotechnol 1999, 17, 969-73.
9. Lukyanov, K. A.; Fradkov, A. F.; Gurskaya, N. G.; Matz, M. V.; Labas, Y. A.; Savitsky, A. P.; Markelov, M. L.; Zaraisky, A. G.; Zhao, X.; Fang, Y.; Tan, W.; Lukyanov, S. A. Natural animal coloration can Be determined by a nonfluorescent green fluorescent protein homolog. J Biol Chem 2000, 275, 25879-82.
10. Gurskaya, N. G.; Fradkov, A. F.; Terskikh, A.; Matz, M. V.; Labas, Y. A.; Martynov, V. I.; Yanushevich, Y. G.; Lukyanov, K. A.; Lukyanov, S, A. GFP-like chromoproteins as a source of far-red fluorescent proteins. FEBS Lett 2001, 507, 16-20.
11. Heim, R.; Prasher, D. C.; Tsien, R. Y. Wavelength mutations and posttranslational autoxidation of green fluorescent protein. Proc Natl Acad Sci U S A 1994, 91, 12501-4.
12. Cubitt, A. B.; Heim, R.; Adams, S. R.; Boyd, A. E.; Gross, L. A.; Tsien, R. Y. Understanding, improving and using green fluorescent proteins. Trends Biochem Sci 1995, 20, 448-55.
13. Reid, B. G.; Flynn, G. C. Chromophore formation in green fluorescent protein. Biochemistry 1997, 36, 6786-91.
14. Biron, K. Fireflies, Dead Fish and a Glowing Bunny: a primer on Bioluminescence. BioTeach Online 2003, 1, 19-26.
15. Davenport, D.; Nicol, J. A. C. Luminescence in Hydromedusae. Proceedings of the Royal Society of London. Series B, Biological Sciences 1955, 144, 399-411.
16. Shimomura, O.; Johnson, F. H.; Saiga, Y. Extraction, purification and properties of aequorin, a bioluminescent protein from the luminous hydromedusan, Aequorea. J Cell Comp Physiol 1962, 59, 223-39.
17. Morise, H.; Shimomura, O.; Johnson, F. H.; Winant, J. Intermolecular energy transfer in the bioluminescent system of Aequorea. Biochemistry 1974, 13, 2656-62.
18. Prendergast, F. G.; Mann, K. G. Chemical and physical properties of aequorin and the green fluorescent protein isolated from Aequorea forskalea. Biochemistry 1978, 17, 3448-53.
19. Prasher, D. C.; Eckenrode, V. K.; Ward, W. W.; Prendergast, F. G.; Cormier, M. J. Primary structure of the Aequorea victoria green-fluorescent protein. Gene 1992, 111, 229-33.
20. Chalfie, M.; Tu, Y.; Euskirchen, G.; Ward, W. W.; Prasher, D. C. Green fluorescent protein as a marker for gene expression. Science 1994, 263, 802-5.
21. Cody, C. W.; Prasher, D. C.; Westler, W. M.; Prendergast, F. G.; Ward, W. W. Chemical structure of the hexapeptide chromophore of the Aequorea green-fluorescent protein. Biochemistry 1993, 32, 1212-8.
22. Ormo, M.; Cubitt, A. B.; Kallio, K.; Gross, L. A.; Tsien, R. Y.; Remington, S. J. Crystal structure of the Aequorea victoria green fluorescent protein. Science 1996, 273, 1392-5.
23. Yang, F.; Moss, L. G.; Phillips, G. N., Jr. The molecular structure of green fluorescent protein. Nat Biotechnol 1996, 14, 1246-51.
24. Tsien, R. Y. The green fluorescent protein. Annu Rev Biochem 1998, 67, 509-44.
25. Kendall, J. M.; Badminton, M. N. Aequorea victoria bioluminescence moves into an exciting new era. Trends Biotechnol 1998, 16, 216-24.
26. Baird, G. S.; Zacharias, D. A.; Tsien, R. Y. Biochemistry, mutagenesis, and oligomerization of DsRed, a red fluorescent protein from coral. Proc Natl Acad Sci U S A 2000, 97, 11984-9.
27. Verkhusha, V. V.; Lukyanov, K. A. The molecular properties and applications of Anthozoa fluorescent proteins and chromoproteins. Nat Biotechnol 2004, 22, 289-96.
28. Sambrook, J.; Russell, D. W. MOLECULAR CLONING A LABORATORY MANUAL. 2 ed.; Cold Spring Harbor Laboratory Press: 1989.
29. Chudakov, D. M.; Lukyanov, S.; Lukyanov, K. A. Fluorescent proteins as a toolkit for in vivo imaging. Trends Biotechnol 2005, 23, 605-13.
30. Licha, K.; Olbrich, C. Optical imaging in drug discovery and diagnostic applications. Adv Drug Deliv Rev 2005, 57, 1087-108.
31. Weissleder, R. Scaling down imaging: molecular mapping of cancer in mice. Nat Rev Cancer 2002, 2, 11-8.
32. Campbell, R. E.; Tour, O.; Palmer, A. E.; Steinbach, P. A.; Baird, G. S.; Zacharias, D. A.; Tsien, R. Y. A monomeric red fluorescent protein. Proc Natl Acad Sci U S A 2002, 99, 7877-82.
33. Yarbrough, D.; Wachter, R. M.; Kallio, K.; Matz, M. V.; Remington, S. J. Refined crystal structure of DsRed, a red fluorescent protein from coral, at 2.0-A resolution. Proc Natl Acad Sci U S A 2001, 98, 462-7.
34. Miyazaki, K. Creating random mutagenesis libraries by megaprimer PCR of whole plasmid (MEGAWHOP). Methods Mol Biol 2003, 231, 23-8.
35. Rao, A.; Luo, C.; Hogan, P. G. Transcription factors of the NFAT family: regulation and function. Annu Rev Immunol 1997, 15, 707-47.

## Claims

1. An isolated fluorescent protein or polypeptide which:
(a) emits green light upon excitation with light in the range of λ 396-490 nm;
(b) has an amino acid sequence which is at least 80%, preferably at least 90%, more preferably at least 95% identical to SEQ ID NO°:1;
(c) in sequence alignment with SEQ ID NO°:1, presents one of the following multiple amino acid substitutions (the residue positions are referred to SEQ ID NO°:1):
viii) S103K
ix) S103K, T159K and F173E
x) S94T, S103K, T159K and F173E
xi) S103K, R150E
xii) S103K, F 173E
xiii) S94T, S103K, R150K, S 171N and K206R
xiv) S94T, S103K, R150I, S171N and K206R

2. A fragment of a protein or a polypeptide as per claim 1, said fragment being water-soluble, retaining the capability of emitting green light upon excitation with light in the range of λ 396-490 nm and presenting at least one of the substitutions indicated in claim 1.

3. A protein or polypeptide according to claim 1, which is selected from the group consisting of SEQ ID NOs:2-8.

4. An antibody able to specifically recognize and bind a fluorescent protein, a polypeptide or a fragment thereof according to claims 1-3.

5. A nucleic acid molecule encoding a protein or polypeptide or fragment thereof according to claims 1-3.

6. A nucleic acid molecule according to claim 5 which is selected from SEQ ID NOs:11-17 or a complementary strand thereof or a sequence hybridizing thereto under stringent conditions.

7. A nucleic acid molecule according to claim 5 or 6, the codon usage of which has been optimized for the expression in a mammalian cell.

8. An expression vector carrying a nucleic acid molecule according to claims 5-6.

9. A prokaryotic or eukaryotic host cell containing a nucleic acid molecule according to claims 5-6 or an expression vector according to claim 8.

10. A method for detecting the fluorescence in a cell, which comprises:
(a) providing a cell expressing a fluorescent protein, polypeptide or fragment thereof according to claims 1-3;
(b) exposing the cells to excitation with light in the range of λ 396-490 nm;
(c) detecting the fluorescence.

11. The use of a fluorescent protein or polypeptide or fragment thereof according to claims 1-3 as intracellular fluorescent marker.

12. The use according to claim 11, for labelling, localizing or detecting a biological molecule or a compound in a cell or cell organelle.

13. The use of a fluorescent protein or polypeptide or fragment thereof according to claims 1-3 for setting up a cell-based or cell-free assay.

14. The use according to claim 13, wherein said assay is carried out in a high throughput format.

15. A kit for use in a biological assay which contains a fluorescent protein or polypeptide or fragment thereof according to claims 1-3 and/or an antibody according to claim 4 and/or a nucleic acid molecule according to claims 5-7 and/or an expression vector according to claim 8, packaged in a suitable container.


SEQUENCE LISTING

<110>  AXXAM S.p.A

<120>  Fluorescent proteins and methods of use thereof

<130>  1808eur

<160>  51

<170>  PatentIn version 3.3

<210>  1
<211>  228
<212>  PRT
<213>  Anemonia sulcata

<400>  1

Met Tyr Pro Ser Ile Lys Glu Thr Met Arg Val Gln Leu Ser Met Glu
1               5                   10                  15

Gly Ser Val Asn Tyr His Ala Phe Lys Cys Thr Gly Lys Gly Glu Gly
            20                  25                  30

Lys Pro Tyr Glu Gly Thr Gln Ser Leu Asn Ile Thr Ile Thr Glu Gly
        35                  40                  45

Gly Pro Leu Pro Phe Ala Phe Asp Ile Leu Ser His Ala Phe Gln Tyr
    50                  55                  60

Gly Ile Lys Val Phe Ala Lys Tyr Pro Lys Glu Ile Pro Asp Phe Phe
65                  70                  75                  80

Lys Gln Ser Leu Pro Gly Gly Phe Ser Trp Glu Arg Val Ser Thr Tyr
                85                  90                  95

Glu Asp Gly Gly Val Leu Ser Ala Thr Gln Glu Thr Ser Leu Gln Gly
            100                 105                 110

Asp Cys Ile Ile Cys Lys Val Lys Val Leu Gly Thr Asn Phe Pro Ala
        115                 120                 125

Asn Gly Pro Val Met Gln Lys Lys Thr Cys Gly Trp Glu Pro Ser Thr
    130                 135                 140

Glu Thr Val Ile Pro Arg Asp Gly Gly Leu Leu Leu Arg Asp Thr Pro
145                 150                 155                 160
Ala Leu Met Leu Ala Asp Gly Gly His Leu Ser Cys Phe Met Glu Thr
                165                 170                 175

Thr Tyr Lys Ser Lys Lys Glu Val Lys Leu Pro Glu Leu His Phe His
            180                 185                 190

His Leu Arg Met Glu Lys Leu Asn Ile Ser Asp Asp Trp Lys Thr Val
        195                 200                 205

Glu Gln His Glu Ser Val Val Ala Ser Tyr Ser Gln Val Pro Ser Lys
    210                 215                 220

Leu Gly His Asn
225

<210>  2
<211>  228
<212>  PRT
<213>  Unknown

<220>
<223>  AsGFP499 mutant

<400>  2

Met Tyr Pro Ser Ile Lys Glu Thr Met Arg Val Gln Leu Ser Met Glu
1               5                   10                  15

Gly Ser Val Asn Tyr His Ala Phe Lys Cys Thr Gly Lys Gly Glu Gly
            20                  25                  30

Lys Pro Tyr Glu Gly Thr Gln Ser Leu Asn Ile Thr Ile Thr Glu Gly
        35                  40                  45

Gly Pro Leu Pro Phe Ala Phe Asp Ile Leu Ser His Ala Phe Gln Tyr
    50                  55                  60

Gly Ile Lys Val Phe Ala Lys Tyr Pro Lys Glu Ile Pro Asp Phe Phe
65                  70                  75                  80

Lys Gln Ser Leu Pro Gly Gly Phe Ser Trp Glu Arg Val Ser Thr Tyr
                85                  90                  95

Glu Asp Gly Gly Val Leu Lys Ala Thr Gln Glu Thr Ser Leu Gln Gly
            100                 105                 110

Asp Cys Ile Ile Cys Lys Val Lys Val Leu Gly Thr Asn Phe Pro Ala
        115                 120                 125

Asn Gly Pro Val Met Gln Lys Lys Thr Cys Gly Trp Glu Pro Ser Thr
    130                 135                 140

Glu Thr Val Ile Pro Arg Asp Gly Gly Leu Leu Leu Arg Asp Thr Pro
145                 150                 155                 160

Ala Leu Met Leu Ala Asp Gly Gly His Leu Ser Cys Phe Met Glu Thr
                165                 170                 175

Thr Tyr Lys Ser Lys Lys Glu Val Lys Leu Pro Glu Leu His Phe His
            180                 185                 190

His Leu Arg Met Glu Lys Leu Asn Ile Ser Asp Asp Trp Lys Thr Val
        195                 200                 205

Glu Gln His Glu Ser Val Val Ala Ser Tyr Ser Gln Val Pro Ser Lys
    210                 215                 220

Leu Gly His Asn
225

<210>  3
<211>  228
<212>  PRT
<213>  Unknown

<220>
<223>  AsGFP499 mutant

<400>  3

Met Tyr Pro Ser Ile Lys Glu Thr Met Arg Val Gln Leu Ser Met Glu
1               5                   10                  15

Gly Ser Val Asn Tyr His Ala Phe Lys Cys Thr Gly Lys Gly Glu Gly
            20                  25                  30

Lys Pro Tyr Glu Gly Thr Gln Ser Leu Asn Ile Thr Ile Thr Glu Gly
        35                  40                  45

Gly Pro Leu Pro Phe Ala Phe Asp Ile Leu Ser His Ala Phe Gln Tyr
    50                  55                  60

Gly Ile Lys Val Phe Ala Lys Tyr Pro Lys Glu Ile Pro Asp Phe Phe
65                  70                  75                  80

Lys Gln Ser Leu Pro Gly Gly Phe Ser Trp Glu Arg Val Ser Thr Tyr
                85                  90                  95

Glu Asp Gly Gly Val Leu Lys Ala Thr Gln Glu Thr Ser Leu Gln Gly
            100                 105                 110

Asp Cys Ile Ile Cys Lys Val Lys Val Leu Gly Thr Asn Phe Pro Ala
        115                 120                 125

Asn Gly Pro Val Met Gln Lys Lys Thr Cys Gly Trp Glu Pro Ser Thr
    130                 135                 140

Glu Thr Val Ile Pro Arg Asp Gly Gly Leu Leu Leu Arg Asp Lys Pro
145                 150                 155                 160

Ala Leu Met Leu Ala Asp Gly Gly His Leu Ser Cys Glu Met Glu Thr
                165                 170                 175

Thr Tyr Lys Ser Lys Lys Glu Val Lys Leu Pro Glu Leu His Phe His
            180                 185                 190

His Leu Arg Met Glu Lys Leu Asn Ile Ser Asp Asp Trp Lys Thr Val
        195                 200                 205

Glu Gln His Glu Ser Val Val Ala Ser Tyr Ser Gln Val Pro Ser Lys
    210                 215                 220

Leu Gly His Asn
225

<210> 4
<211> 228
<212> PRT
<213> Unknown

<220>
<223> AsGFP499 mutant

<400> 4

Met Tyr Pro Ser Ile Lys Glu Thr Met Arg Val Gln Leu Ser Met Glu
1               5                   10                  15

Gly Ser Val Asn Tyr His Ala Phe Lys Cys Thr Gly Lys Gly Glu Gly
            20                  25                  30

Lys Pro Tyr Glu Gly Thr Gln Ser Leu Asn Ile Thr Ile Thr Glu Gly
        35                  40                  45

Gly Pro Leu Pro Phe Ala Phe Asp Ile Leu Ser His Ala Phe Gln Tyr
    50                  55                  60

Gly Ile Lys Val Phe Ala Lys Tyr Pro Lys Glu Ile Pro Asp Phe Phe
65                  70                  75                  80

Lys Gln Ser Leu Pro Gly Gly Phe Ser Trp Glu Arg Val Thr Thr Tyr
                85                  90                  95

Glu Asp Gly Gly Val Leu Lys Ala Thr Gln Glu Thr Ser Leu Gln Gly
            100                 105                 110

Asp Cys Ile Ile Cys Lys Val Lys Val Leu Gly Thr Asn Phe Pro Ala
        115                 120                 125

Asn Gly Pro Val Met Gln Lys Lys Thr Cys Gly Trp Glu Pro Ser Thr
    130                 135                 140

Glu Thr Val Ile Pro Arg Asp Gly Gly Leu Leu Leu Arg Asp Lys Pro
145                 150                 155                 160

Ala Leu Met Leu Ala Asp Gly Gly His Leu Ser Cys Glu Met Glu Thr
                165                 170                 175

Thr Tyr Lys Ser Lys Lys Glu Val Lys Leu Pro Glu Leu His Phe His
            180                 185                 190

His Leu Arg Met Glu Lys Leu Asn Ile Ser Asp Asp Trp Lys Thr Val
        195                 200                 205

Glu Gln His Glu Ser Val Val Ala Ser Tyr Ser Gln Val Pro Ser Lys
    210                 215                 220

Leu Gly His Asn
225

<210>  5
<211>  228
<212>  PRT
<213>  Unknown

<220>
<223>  AsGFP499 mutant

<400>  5

Met Tyr Pro Ser Ile Lys Glu Thr Met Arg Val Gln Leu Ser Met Glu
1               5                   10                  15

Gly Ser Val Asn Tyr His Ala Phe Lys Cys Thr Gly Lys Gly Glu Gly
            20                  25                  30

Lys Pro Tyr Glu Gly Thr Gln Ser Leu Asn Ile Thr Ile Thr Glu Gly
        35                  40                  45

Gly Pro Leu Pro Phe Ala Phe Asp Ile Leu Ser His Ala Phe Gln Tyr
    50                  55                  60

Gly Ile Lys Val Phe Ala Lys Tyr Pro Lys Glu Ile Pro Asp Phe Phe
65                  70                  75                  80

Lys Gln Ser Leu Pro Gly Gly Phe Ser Trp Glu Arg Val Ser Thr Tyr
                85                  90                  95

Glu Asp Gly Gly Val Leu Lys Ala Thr Gln Glu Thr Ser Leu Gln Gly
            100                 105                 110

Asp Cys Ile Ile Cys Lys Val Lys Val Leu Gly Thr Asn Phe Pro Ala
        115                 120                 125

Asn Gly Pro Val Met Gln Lys Lys Thr Cys Gly Trp Glu Pro Ser Thr
    130                 135                 140

Glu Thr Val Ile Pro Glu Asp Gly Gly Leu Leu Leu Arg Asp Thr Pro
145                 150                 155                 160

Ala Leu Met Leu Ala Asp Gly Gly His Leu Ser Cys Phe Met Glu Thr
                165                 170                 175

Thr Tyr Lys Ser Lys Lys Glu Val Lys Leu Pro Glu Leu His Phe His
            180                 185                 190

His Leu Arg Met Glu Lys Leu Asn Ile Ser Asp Asp Trp Lys Thr Val
        195                 200                 205

Glu Gln His Glu Ser Val Val Ala Ser Tyr Ser Gln Val Pro Ser Lys
    210                 215                 220

Leu Gly His Asn
225

<210>  6
<211>  228
<212>  PRT
<213>  Unknown

<220>
<223>  AsGFP499 mutant

<400>  6

Met Tyr Pro Ser Ile Lys Glu Thr Met Arg Val Gln Leu Ser Met Glu
1               5                   10                  15

Gly Ser Val Asn Tyr His Ala Phe Lys Cys Thr Gly Lys Gly Glu Gly
            20                  25                  30

Lys Pro Tyr Glu Gly Thr Gln Ser Leu Asn Ile Thr Ile Thr Glu Gly
        35                  40                  45

Gly Pro Leu Pro Phe Ala Phe Asp Ile Leu Ser His Ala Phe Gln Tyr
    50                  55                  60

Gly Ile Lys Val Phe Ala Lys Tyr Pro Lys Glu Ile Pro Asp Phe Phe
65                  70                  75                  80

Lys Gln Ser Leu Pro Gly Gly Phe Ser Trp Glu Arg Val Ser Thr Tyr
                85                  90                  95

Glu Asp Gly Gly Val Leu Lys Ala Thr Gln Glu Thr Ser Leu Gln Gly
            100                 105                 110

Asp Cys Ile Ile Cys Lys Val Lys Val Leu Gly Thr Asn Phe Pro Ala
        115                 120                 125

Asn Gly Pro Val Met Gln Lys Lys Thr Cys Gly Trp Glu Pro Ser Thr
    130                 135                 140

Glu Thr Val Ile Pro Arg Asp Gly Gly Leu Leu Leu Arg Asp Thr Pro
145                 150                 155                 160

Ala Leu Met Leu Ala Asp Gly Gly His Leu Ser Cys Glu Met Glu Thr
                165                 170                 175

Thr Tyr Lys Ser Lys Lys Glu Val Lys Leu Pro Glu Leu His Phe His
            180                 185                 190

His Leu Arg Met Glu Lys Leu Asn Ile Ser Asp Asp Trp Lys Thr Val
        195                 200                 205

Glu Gln His Glu Ser Val Val Ala Ser Tyr Ser Gln Val Pro Ser Lys
    210                 215                 220

Leu Gly His Asn
225

<210> 7
<211> 228
<212> PRT
<213> Unknown

<220>
<223> AsGFP499 mutant

<400> 7

Met Tyr Pro Ser Ile Lys Glu Thr Met Arg Val Gln Leu Ser Met Glu
1               5                   10                  15

Gly Ser Val Asn Tyr His Ala Phe Lys Cys Thr Gly Lys Gly Glu Gly
            20                  25                  30

Lys Pro Tyr Glu Gly Thr Gln Ser Leu Asn Ile Thr Ile Thr Glu Gly
        35                  40                  45

Gly Pro Leu Pro Phe Ala Phe Asp Ile Leu Ser His Ala Phe Gln Tyr
    50                  55                  60

Gly Ile Lys Val Phe Ala Lys Tyr Pro Lys Glu Ile Pro Asp Phe Phe
65                  70                  75                  80

Lys Gln Ser Leu Pro Gly Gly Phe Ser Trp Glu Arg Val Thr Thr Tyr
                85                  90                  95

Glu Asp Gly Gly Val Leu Lys Ala Thr Gln Glu Thr Ser Leu Gln Gly
            100                 105                 110

Asp Cys Ile Ile Cys Lys Val Lys Val Leu Gly Thr Asn Phe Pro Ala
        115                 120                 125

Asn Gly Pro Val Met Gln Lys Lys Thr Cys Gly Trp Glu Pro Ser Thr
    130                 135                 140

Glu Thr Val Ile Pro Lys Asp Gly Gly Leu Leu Leu Arg Asp Thr Pro
145                 150                 155                 160

Ala Leu Met Leu Ala Asp Gly Gly His Leu Asn Cys Phe Met Glu Thr
                165                 170                 175

Thr Tyr Lys Ser Lys Lys Glu Val Lys Leu Pro Glu Leu His Phe His
            180                 185                 190

His Leu Arg Met Glu Lys Leu Asn Ile Ser Asp Asp Trp Arg Thr Val
        195                 200                 205

Glu Gln His Glu Ser Val Val Ala Ser Tyr Ser Gln Val Pro Ser Lys
    210                 215                 220

Leu Gly His Asn
225

<210> 8

<211>  228
<212>  PRT
<213>  Unknown

<220>
<223>  AsGFP499 mutant

<400>  8

Met Tyr Pro Ser Ile Lys Glu Thr Met Arg Val Gln Leu Ser Met Glu
1               5                   10                  15

Gly Ser Val Asn Tyr His Ala Phe Lys Cys Thr Gly Lys Gly Glu Gly
            20                  25                  30

Lys Pro Tyr Glu Gly Thr Gln Ser Leu Asn Ile Thr Ile Thr Glu Gly
        35                  40                  45

Gly Pro Leu Pro Phe Ala Phe Asp Ile Leu Ser His Ala Phe Gln Tyr
    50                  55                  60

Gly Ile Lys Val Phe Ala Lys Tyr Pro Lys Glu Ile Pro Asp Phe Phe
65                  70                  75                  80

Lys Gln Ser Leu Pro Gly Gly Phe Ser Trp Glu Arg Val Thr Thr Tyr
                85                  90                  95

Glu Asp Gly Gly Val Leu Lys Ala Thr Gln Glu Thr Ser Leu Gln Gly
            100                 105                 110

Asp Cys Ile Ile Cys Lys Val Lys Val Leu Gly Thr Asn Phe Pro Ala
        115                 120                 125

Asn Gly Pro Val Met Gln Lys Lys Thr Cys Gly Trp Glu Pro Ser Thr
    130                 135                 140

Glu Thr Val Ile Pro Ile Asp Gly Gly Leu Leu Leu Arg Asp Thr Pro
145                 150                 155                 160

Ala Leu Met Leu Ala Asp Gly Gly His Leu Asn Cys Phe Met Glu Thr
                165                 170                 175

Thr Tyr Lys Ser Lys Lys Glu Val Lys Leu Pro Glu Leu His Phe His
            180                 185                 190

His Leu Arg Met Glu Lys Leu Asn Ile Ser Asp Asp Trp Arg Thr Val
        195                 200                 205

Glu Gln His Glu Ser Val Val Ala Ser Tyr Ser Gln Val Pro Ser Lys
    210                 215                 220

Leu Gly His Asn
225

<210> 9
<211> 687
<212> DNA
<213> Unknown

<220>
<223> wt AsGFP499 coding sequence adapted to mammalian codon usage

<400> 9
atgtacccca gcatcaagga gaccatgcgg gtgcagctga gcatggaggg cagcgtgaac 60

taccacgcct tcaagtgcac cggcaagggc gagggcaagc cctacgaggg cacccagagc 120

ctgaacatca ccatcaccga gggcggaccc ctgcctttcg ccttcgacat cctgagccac 180

gccttccagt acggcatcaa ggtgttcgcc aagtacccca aggagatccc cgacttcttc 240

aagcagagcc tgcctggcgg cttcagctgg gagagagtga gcacctacga ggacggcggc 300

gtgctgagcg ccacccagga gaccagcctg cagggcgatt gcatcatctg caaggtgaag 360

gtgctgggca ccaactttcc tgccaacggc cccgtgatgc agaagaaaac ctgcggctgg 420

gagcccagca ccgagaccgt gatccccaga gatggcggcc tgctgctgag agacacccct 480

gccctcatgc tggccgacgg cggccacctg agctgcttta tggagaccac ctacaagagc 540

aagaaggagg tgaagctgcc cgagctgcac tttcaccacc tgcggatgga gaagctgaac 600

atcagcgacg actggaaaac cgtggagcag cacgagagcg tggtggccag ctacagccag 660

gtgccaagca agctgggcca caattga 687

<210> 10
<211> 687
<212> DNA
<213> Anemonia sulcata

<400> 10
atgtatcctt ccatcaagga aaccatgcgc gttcagcttt ctatggaggg tagtgttaac 60

taccacgcct tcaagtgcac tggaaaagga gagggaaaac catacgaagg cacccaaagc 120

ctgaatatta caataactga aggaggtcct ctgccatttg cttttgacat tctgtcacac 180

gcctttcagt atggcatcaa ggtcttcgcc aagtacccca aagaaattcc tgacttcttt 240

aagcagtctc tacctggtgg tttttcttgg gaaagagtaa gcacctatga agatggagga 300

gtgctttcag ctacccaaga aacaagtttg cagggtgatt gcatcatctg caaagttaaa 360

gtccttggca ccaattttcc cgcaaacggt ccagtgatgc aaaagaagac ctgtggatgg 420

gagccatcaa ctgaaacagt catcccacga gatggtggac ttctgcttcg cgataccccc 480

gcacttatgc tggctgacgg aggtcatctt tcttgcttca tggaaacaac ttacaagtcg 540

aagaaagagg taaagcttcc agaacttcac tttcatcatt tgcgtatgga aaagctgaac 600

ataagtgacg attggaagac cgttgagcag cacgagtctg tggtggctag ctactcccaa 660

gtgccttcga aattaggaca taactga 687

<210> 11
<211> 687
<212> DNA
<213> Unknown

<220>
<223> mutagenized AsGFP499 coding sequence

<400> 11
atgtacccca gcatcaagga gaccatgcgg gtgcagctga gcatggaggg cagcgtgaac 60

taccacgcct tcaagtgcac cggcaagggc gagggcaagc cctacgaggg cacccagagc 120

ctgaacatca ccatcaccga gggcggaccc ctgcctttcg ccttcgacat cctgagccac 180

gccttccagt acggcatcaa ggtgttcgcc aagtacccca aggagatccc cgacttcttc 240

aagcagagcc tgcctggcgg cttcagctgg gagagagtga gcacctacga ggacggcggc 300

gtgctgaagg ccacccagga gaccagcctg cagggcgatt gcatcatctg caaggtgaag 360

gtgctgggca ccaactttcc tgccaacggc cccgtgatgc agaagaaaac ctgcggctgg 420

gagcccagca ccgagaccgt gatccccaga gatggcggcc tgctgctgag agacacccct 480

gccctcatgc tggccgacgg cggccacctg agctgcttta tggagaccac ctacaagagc 540

aagaaggagg tgaagctgcc cgagctgcac tttcaccacc tgcggatgga gaagctgaac 600

atcagcgacg actggaaaac cgtggagcag cacgagagcg tggtggccag ctacagccag 660

gtgccaagca agctgggcca caattga 687

<210> 12
<211> 687
<212> DNA
<213> Unknown

<220>
<223> mutagenized AsGFP499 coding sequence

<400> 12
atgtacccca gcatcaagga gaccatgcgg gtgcagctga gcatggaggg cagcgtgaac 60

taccacgcct tcaagtgcac cggcaagggc gagggcaagc cctacgaggg cacccagagc 120

ctgaacatca ccatcaccga gggcggaccc ctgcctttcg ccttcgacat cctgagccac 180

gccttccagt acggcatcaa ggtgttcgcc aagtacccca aggagatccc cgacttcttc 240

aagcagagcc tgcctggcgg cttcagctgg gagagagtga gcacctacga ggacggcggc 300

gtgctgaagg ccacccagga gaccagcctg cagggcgatt gcatcatctg caaggtgaag 360

gtgctgggca ccaactttcc tgccaacggc cccgtgatgc agaagaaaac ctgcggctgg 420

gagcccagca ccgagaccgt gatccccaga gatggcggcc tgctgcttag agacaaacct 480

gccctcatgc tggccgacgg cggccacctg agctgcgaga tggagaccac ctacaagagc 540

aagaaggagg tgaagctgcc cgagctgcac tttcaccacc tgcggatgga gaagctgaac 600

atcagcgacg actggaaaac cgtggagcag cacgagagcg tggtggccag ctacagccag 660

gtgccaagca agctgggcca caattga 687

<210> 13
<211> 687
<212> DNA
<213> Unknown

<220>
<223> mutagenized AsGFP499 coding sequence

<400> 13
atgtacccca gcatcaagga gaccatgcgg gtgcagctga gcatggaggg cagcgtgaac 60

taccacgcct tcaagtgcac cggcaagggc gagggcaagc cctacgaggg cacccagagc 120

ctgaacatca ccatcaccga gggcggaccc ctgcctttcg ccttcgacat cctgagccac 180

gccttccagt acggcatcaa ggtgttcgcc aagtacccca aggagatccc cgacttcttc 240

aagcagagcc tgcctggcgg cttcagctgg gagagagtga ccacctacga ggacggcggc 300

gtgctgaagg ccacccagga gaccagcctg cagggcgatt gcatcatctg caaggtgaag 360

gtgctgggca ccaactttcc tgccaacggc cccgtgatgc agaagaaaac ctgcggctgg 420

gagcccagca ccgagaccgt gatccccaga gatggcggcc tgctgcttag agacaaacct 480

gccctcatgc tggccgacgg cggccacctg agctgcgaga tggagaccac ctacaagagc 540

aagaaggagg tgaagctgcc cgagctgcac tttcaccacc tgcggatgga gaagctgaac 600

atcagcgacg actggaaaac cgtggagcag cacgagagcg tggtggccag ctacagccag 660

gtgccaagca agctgggcca caattga 687

<210> 14
<211> 687
<212> DNA
<213> Unknown

<220>
<223> mutagenized AsGFP499 coding sequence

<400> 14
atgtacccca gcatcaagga gaccatgcgg gtgcagctga gcatggaggg cagcgtgaac 60

taccacgcct tcaagtgcac cggcaagggc gagggcaagc cctacgaggg cacccagagc 120

ctgaacatca ccatcaccga gggcggaccc ctgcctttcg ccttcgacat cctgagccac 180

gccttccagt acggcatcaa ggtgttcgcc aagtacccca aggagatccc cgacttcttc 240

aagcagagcc tgcctggcgg cttcagctgg gagagagtga gcacctacga ggacggcggc 300

gtgctgaagg ccacccagga gaccagcctg cagggcgatt gcatcatctg caaggtgaag 360

gtgctgggca ccaactttcc tgccaacggc cccgtgatgc agaagaaaac ctgcggctgg 420

gagcccagca ccgagaccgt gatccccgaa gatggcggcc tgctgctgag agacacccct 480

gccctcatgc tggccgacgg cggccacctg agctgcttta tggagaccac ctacaagagc 540

aagaaggagg tgaagctgcc cgagctgcac tttcaccacc tgcggatgga gaagctgaac 600

atcagcgacg actggaaaac cgtggagcag cacgagagcg tggtggccag ctacagccag 660

gtgccaagca agctgggcca caattga 687

<210> 15
<211> 687
<212> DNA
<213> Unknown

<220>
<223> mutagenized AsGFP499 coding sequence

<400> 15
atgtacccca gcatcaagga gaccatgcgg gtgcagctga gcatggaggg cagcgtgaac 60

taccacgcct tcaagtgcac cggcaagggc gagggcaagc cctacgaggg cacccagagc 120

ctgaacatca ccatcaccga gggcggaccc ctgcctttcg ccttcgacat cctgagccac 180

gccttccagt acggcatcaa ggtgttcgcc aagtacccca aggagatccc cgacttcttc 240

aagcagagcc tgcctggcgg cttcagctgg gagagagtga gcacctacga ggacggcggc 300

gtgctgaagg ccacccagga gaccagcctg cagggcgatt gcatcatctg caaggtgaag 360

gtgctgggca ccaactttcc tgccaacggc cccgtgatgc agaagaaaac ctgcggctgg 420

gagcccagca ccgagaccgt gatccccaga gatggcggcc tgctgctgag agacacccct 480

gccctcatgc tggccgacgg cggccacctg agctgcgaga tggagaccac ctacaagagc 540

aagaaggagg tgaagctgcc cgagctgcac tttcaccacc tgcggatgga gaagctgaac 600

atcagcgacg actggaaaac cgtggagcag cacgagagcg tggtggccag ctacagccag 660

gtgccaagca agctgggcca caattga 687

<210> 16
<211> 687
<212> DNA
<213> Unknown
<220>
<223> mutagenized AsGFP499 coding sequence

<400> 16
atgtacccca gcatcaagga gaccatgcgg gtgcagctga gcatggaggg cagcgtgaac 60

taccacgcct tcaagtgcac cggcaagggc gagggcaagc cctacgaggg cacccagagc 120

ctgaacatca ccatcaccga gggcggaccc ctgcctttcg ccttcgacat cctgagccac 180

gccttccagt acggcatcaa ggtgttcgcc aagtacccca aggagatccc cgacttcttc 240

aagcagagcc tgcctggcgg cttcagctgg gagagagtga ccacctacga ggacggcggc 300

gtgctgaagg ccacccagga gaccagcctg cagggcgatt gcatcatctg caaggtgaag 360

gtgctgggca ccaactttcc tgccaacggc cccgtgatgc agaagaaaac ctgcggctgg 420

gagcccagca ctgagaccgt gatccccaaa gatggcggcc tgctgctgag agacacccct 480

gccctcatgc tggccgacgg cggtcacctg aactgcttta tggagaccac ctataagagc 540

aagaaggagg tgaagctgcc cgagctgcac tttcaccacc tgcggatgga gaagctgaac 600

atcagcgacg actggagaac cgtggagcag catgagagcg tggtggccag ctacagccag 660

gtgccaagta agctgggcca caattga 687

<210> 17
<211> 687
<212> DNA
<213> Unknown

<220>
<223> mutagenized AsGFP499 coding sequence

<400> 17
atgtacccca gcatcaagga gaccatgcgg gtgcagctga gcatggaggg cagcgtgaac 60

taccacgcct tcaagtgcac cggcaagggc gagggcaagc cctacgaggg cacccagagc 120

ctgaacatca ccatcaccga gggcggaccc ctgcctttcg ccttcgacat cctgagccac 180

gccttccagt acggcatcaa ggtgttcgcc aagtacccca aggagatccc cgacttcttc 240

aagcagagcc tgcctggcgg cttcagctgg gagagagtga ccacctacga ggacggcggc 300

gtgctgaagg ccacccagga gaccagcctg cagggcgatt gcatcatctg caaggtgaag 360

gtgctgggca ccaactttcc tgccaacggc cccgtgatgc agaagaaaac ctgcggctgg 420

gagcccagca ctgagaccgt gatccccata gatggcggcc tgctgctgag agacacccct 480

gccctcatgc tggccgacgg cggtcacctg aactgcttta tggagaccac ctataagagc 540

aagaaggagg tgaagctgcc cgagctgcac tttcaccacc tgcggatgga gaagctgaac 600

atcagcgacg actggagaac cgtggagcag catgagagcg tggtggccag ctacagccag 660

gtgccaagca agctggggca caattga 687

<210> 18
<211> 28
<212> DNA
<213> Unknown

<220>
<223>  synthetic oligonucleotide

<400>  18
cggcagccat atgtacccca gcatcaag                                          28


<210>  19
<211>  26
<212>  DNA
<213>  Unknown

<220>
<223>  synthetic oligonucleotide

<400>  19
gcggatcctt atcaattgtg gcccag                                            26


<210>  20
<211>  25
<212>  DNA
<213>  Unknown

<220>
<223>  synthetic oligonucleotide

<400>  20
taaggatccg atgtacccca gcatc                                             25


<210>  21
<211>  24
<212>  DNA
<213>  Unknown

<220>
<223>  synthetic oligonucleotide

<400>  21
catggtacct caattgtggc ccag                                              24


<210>  22
<211>  24
<212>  DNA
<213>  Unknown
<220>
<223>  synthetic oligonucleotide

<400>  22
cttggtacca tgtaccccag catc                                              24


<210>  23
<211>  35
<212>  DNA
<213>  Unknown

<220>
<223>  synthetic oligonucleotide

<400> 23
tagagtcgcg gccgctcaat tgtgccccag cttgc 35

<210> 24
<211> 40
<212> DNA
<213> Unknown

<220>
<223> synthetic oligonucleotide

<400> 24
ccgggatcca ccggtcgcca ccatgtaccc cagcatcaag 40

<210> 25
<211> 35
<212> DNA
<213> Unknown

<220>
<223> synthetic oligonucleotide

<400> 25
tagagtcgcg gccgctcaat tgtgccccag cttgc 35

<210> 26
<211> 21
<212> DNA
<213> Unknown

<220>
<223> synthetic oligonucleotide

<400> 26
taccccagca tcaaggagac c 21

<210> 27
<211> 35
<212> DNA
<213> Unknown

<220>
<223> synthetic oligonucleotide

<400> 27
tagagtcgcg gccgctcaat tgtgccccag cttgc 35

<210> 28
<211> 27
<212> DNA
<213> Unknown

<220>
<223> synthetic oligonucleotide

<400> 28
gaggtaccat gccaagcacc agctttc 27

<210> 29
<211> 27
<212> DNA
<213> Unknown

<220>
<223> synthetic oligonucleotide

<400> 29
ctggatcccg gaaaaagcac cccacgc 27


<210> 30
<211> 26
<212> DNA
<213> Unknown

<220>
<223> synthetic oligonucleotide

<400> 30
gggagagagt gaccacctac gaggac 26


<210> 31
<211> 26
<212> DNA
<213> Unknown

<220>
<223> synthetic oligonucleotide

<400> 31
gtcctcgtag gtggtcactc tctccc 26

<210> 32
<211> 29
<212> DNA
<213> Unknown

<220>
<223> synthetic oligonucleotide

<400> 32
gagtgagcac ctacggcgac ggcggcgtg 29


<210> 33
<211> 29
<212> DNA
<213> Unknown

<220>
<223> synthetic oligonucleotide

<400> 33
cacgccgccg tcgccgtagg tgctcactc 29


<210> 34
<211> 27
<212> DNA

<213>  Unknown

<220>
<223>  synthetic oligonucleotide

<400>  34
ggcggcgtgc tgaaggccac ccaggag                                          27


<210>  35
<211>  27
<212>  DNA
<213>  Unknown

<220>
<223>  synthetic oligonucleotide

<400>  35
ctcctgggtg gccttcagca cgccgcc                                          27


<210>  36
<211>  27
<212>  DNA
<213>  Unknown

<220>
<223>  synthetic oligonucleotide

<400>  36
accgtgatcc ccgaggatgg cggcctg                                          27


<210>  37
<211>  27
<212>  DNA
<213>  Unknown

<220>
<223>  synthetic oligonucleotide

<400>  37
caggccgcca tcctcgggga tcacggt                                          27



<210>  38
<211>  26
<212>  DNA
<213>  Unknown

<220>
<223>  synthetic oligonucleotide

<400>  38
gcctgctgct gggcgacacc cctgcc                                           26


<210>  39
<211>  26
<212>  DNA
<213>  Unknown

<220>
<223> synthetic oligonucleotide

<400> 39
ggcaggggtg tcgcccagca gcaggc 26

<210> 40
<211> 30
<212> DNA
<213> Unknown

<220>
<223> synthetic oligonucleotide

<400> 40
tgctgcttag agacaaacct gccctcatgc 30

<210> 41
<211> 30
<212> DNA
<213> Unknown

<220>
<223> synthetic oligonucleotide

<400> 41
gcatgagggc aggtttgtct ctaagcagca 30

<210> 42
<211> 30
<212> DNA
<213> Unknown

<220>
<223> synthetic oligonucleotide

<400> 42
tgctgcttag agacgcacct gccctcatgc 30

<210> 43
<211> 30
<212> DNA
<213> Unknown

<220>
<223> synthetic oligonucleotide

<400> 43
gcatgagggc aggtgcgtct ctaagcagca 30

<210> 44
<211> 30
<212> DNA
<213> Unknown

<220>
<223> synthetic oligonucleotide

<400>  44
cggcggccac ctagcatgct ttatggagac                                        30

<210>  45
<211>  30
<212>  DNA
<213>  Unknown

<220>
<223>  synthetic oligonucleotide

<400>  45
gtctccataa agcatgctag gtggccgccg                                        30

<210>  46
<211>  27
<212>  DNA
<213>  Unknown

<220>
<223>  synthetic oligonucleotide

<400>  46
cacctgagct gccacatgga gaccacc                                           27

<210>  47
<211>  27
<212>  DNA
<213>  Unknown

<220>
<223>  synthetic oligonucleotide

<400>  47
ggtggtctcc atgtggcagc tcaggtg                                           27

<210>  48
<211>  27
<212>  DNA
<213>  Unknown

<220>
<223>  synthetic oligonucleotide

<400>  48
cacctgagct gcgccatgga gaccacc                                           27

<210>  49
<211>  27
<212>  DNA
<213>  Unknown

<220>
<223>  synthetic oligonucleotide

<400>  49
ggtggtctcc atggcgcagc tcaggtg                                           27

<210>  50
<211>  27
<212>  DNA
<213>  Unknown

<220>
<223>  synthetic oligonucleotide

<400>  50
cacctgagct gcgagatgga gaccacc                                          27


<210>  51
<211>  27
<212>  DNA
<213>  Unknown

<220>
<223>  synthetic oligonucleotide

<400>  51
ggtggtctcc atctcgcagc tcaggtg                                          27